# EUROPEAN PATENT APPLICATION

(11) **EP 4 000 606 A1**
(43) Date of publication of application: **25.05.2022**
(21) Application number: 20208428.1
(22) Date of filing: 18.11.2020
(51) Int. Cl.: A61K 31/138, A61K 31/519, A61K 31/69, A61P 31/14

(54) **COMPOUNDS FOR CORONAVIRUS INFECTION TREATMENT AND/OR PREVENTION**

(71) Applicant: Fraunhofer-Gesellschaft zur Förderung der angewandten Forschung e.V., 80686 München (DE)
(72) Inventor: The designation of the inventor has not yet been filed
(74) Representative: Altmann Stößel Dick Patentanwälte PartG mbB

(57) **Abstract**

The present invention relates to a compound selected from the compounds indicated in Table 1 and pharmaceutically acceptable salts and prodrugs thereof for use in treating and/or preventing a coronavirus infection in a subject; and to compositions, methods, kits, and devices related thereto.

## Description

The present invention relates to a compound selected from the compounds indicated in Table 1 and pharmaceutically acceptable salts and prodrugs thereof for use in treating and/or preventing a coronavirus infection in a subject; and to compositions, methods, kits, and devices related thereto.

Coronaviruses (Covs) are a large family of viruses belonging to the family Coronaviridae. The limited number of coronaviruses known to be circulating in humans were considered to cause mild infections and they were regarded in the past as relatively harmless respiratory human pathogens. However, in the last years the emergence of the severe acute respiratory syndrome coronavirus (SARS-CoV) and the Middle East Respiratory Syndrome (MERS) virus revealed that some coronaviruses can cause severe and sometimes fatal respiratory tract infections in humans (Pereira, H et al., 1989 Coronaviridae. Andrewes 'Viruses of Vertebrates, 5th ed., pp. 42-57; Holmes, K.V. et al., 1996. Virology 1, 1075-1093). The first known case of SARS-CoV occurred in Foshan, China in November 2002 and new cases emerged in mainland China in February 2003. The first emergence of MERS-CoV occurred in June 2012 in Saudi Arabia. These events demonstrated that the threats of CoVs should not be underestimated and that it is of paramount importance to advance the knowledge on the replication of these viruses and their interactions with the hosts to develop treatments and vaccines.

In December 2019, atypical pneumonia cases emerged in China and the cause was identified as being a novel coronavirus named SARS-CoV-2 by the WHO. Investigations of the epidemiological and clinical characteristics, and outcomes of patients infected by SARS-CoV-2 demonstrated that the infection caused clusters of severe respiratory illness similar to the known SARS-CoV. Furthermore, it was demonstrated that the SARS-CoV-2 can cause severe illness in some patients, even though initially it did not transmit readily between people. However, more recent epidemiological data suggest the new virus has undergone human host adaptation/evolution and has become more efficient in human-to-human transmission. Analysis of SARS-CoV-2 genome sequences obtained from patients during the beginning of the outbreak demonstrated that they are almost identical to each other and share 79.5% sequence identity to SARS-CoV. Furthermore, the SARS-CoV-2 is 96% identical at the whole genome level to a bat coronavirus. Phylogenetic analysis of CoVs of different species indicated that SARS-CoV-2 could have originated from Chinese horseshoe bats, but the intermediate transmission vehicle has not yet been identified (Forster et al., PNAS 2020 117 (17) 9241-9243). According to this study, SARS-CoV-2 belongs to a novel type of bat coronavirus owing to a high degree of variation from the human SARS virus. SARS-CoV-2 is the seventh member of the family of CoVs that infect humans.

Like SARS-CoV, SARS-CoV-2 enters target cells through an endosomal pathway and also uses the same cell entry receptor, Angiotensin-converting enzyme II (ACE2). Effective therapeutic targets in SARS-CoV and several other CoVs are replication-related enzymes, such as proteases (Wu et al., Acta Pharm Sin B. 2020; 10(5): 766-788). Drugs that inhibit conserved proteases are capable of preventing replication and proliferation of the virus by interfering with the post-translational processing of essential viral polypeptides. They can also reduce the risk of mutation-mediated drug resistance. This was the case for the SARS-CoV, as inhibitors targeting the main protease involved in replication and proliferation were the most effective means to alleviate the epidemic.

However, the design and development of new drugs is a lengthy process that is not thus adequate to face the emergency of the immediate global challenge of COVID-19 outbreak. An alternative, more efficient approach is the repurposing of drugs already tested as safe in man or approved for different therapeutic applications. This is a rapid response solution, since the pharmacokinetic, toxicological, and manufacturing data for the drugs are already available, thus allowing immediate application in clinical setting. (O'Conner and Roth, Nat Rev Drug Disc 2005: 4:1005-1014; Anand et al., Science 2003;300(5626): 1763-7). Therefore, computational drug repurposing is an effective approach to rapidly identify and select drugs safe in man that are promising candidates in the treatment of SARS-CoV-2.

In accordance, the present invention relates to a compound selected from the compounds indicated in Table 1 and pharmaceutically acceptable salts and prodrugs thereof for use in treating and/or preventing a coronavirus infection in a subject.

In general, terms used herein are to be given their ordinary and customary meaning to a person of ordinary skill in the art and, unless indicated otherwise, are not to be limited to a special or customized meaning. As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements. Also, as is understood by the skilled person, the expressions "comprising a" and "comprising an" preferably refer to "comprising one or more", i.e. are equivalent to "comprising at least one".

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

As used herein, the term "standard conditions", if not otherwise noted, relates to IUPAC standard ambient temperature and pressure (SATP) conditions, i.e. preferably, a temperature of 25°C and an absolute pressure of 100 kPa; also preferably, standard conditions include a pH of 7. Moreover, if not otherwise indicated, the term "about" relates to the indicated value with the commonly accepted technical precision in the relevant field, preferably relates to the indicated value ± 20%, more preferably ± 10%, most preferably ± 5%. Further, the term "essentially" indicates that deviations having influence on the indicated result or use are absent, i.e. potential deviations do not cause the indicated result to deviate by more than ± 20%, more preferably ± 10%, most preferably ± 5%. Thus, "consisting essentially of" means including the components specified but excluding other components except for materials present as impurities, unavoidable materials present as a result of processes used to provide the components, and components added for a purpose other than achieving the technical effect of the invention. For example, a composition defined using the phrase "consisting essentially of" encompasses any known acceptable additive, excipient, diluent, carrier, and the like. Preferably, a composition consisting essentially of a set of components will comprise less than 5% by weight, more preferably less than 3% by weight, even more preferably less than 1% by weight, most preferably less than 0.1% by weight of non-specified component(s).

The term "compound" is understood by the skilled person and relates to a chemical compound having a structure as specified herein in Table 1. As the skilled person understands, the compound may also be a salt or a prodrug of the specifically indicated chemical compound. Thus, the compounds may, as the case may be, be used as free acid, free base, or a salt thereof; the skilled person preferably selects a pharmaceutically compatible acid, base, or salt of the compound as appropriate for the intended use and/or mode or administration. In the case of optically active compounds, the compound is an isomer, preferably the isomer, used in at least one clinical trial and, optionally, approved for medical use. Thus, the compounds are well known approved drugs or clinical candidates for different therapeutic indications with "safe in man" trials completed. As referred to herein, the compound is a compound as indicated in Table 1 or a pharmaceutically acceptable salt thereof. Preferably also included are prodrugs of the aforesaid compounds. the compound may be in an anhydrous or a hydrate form.

**Table 1: Compound list.**

| **N.** | **Chemical structure** | **Generic/Drug Name** | **Chemical Name** |
|---|---|---|---|
| 1 | 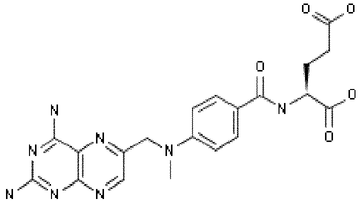 | METHOTREXATE | N-[4-[N-(2,4-Diaminopteridin-6-ylmethyl)-N-methylamino]benzoyl]-L-glutamic acid |
| 2 | 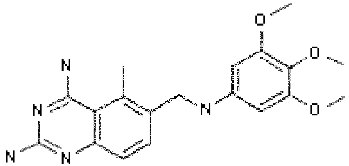 | Trimetrexate | 2,4-Diamino-5-methyl-6-[(3,4,5-trimethoxyanilino)methyl]quinaz oline 5-Methyl-6-[[(3,4,5-trimetoxyphenyl)amino]methyl]-2,4-quinazolinediamine |
| 3 | 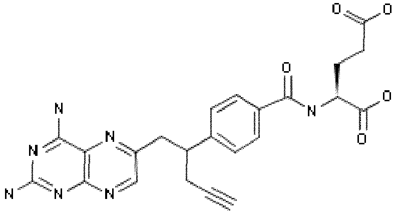 | Pralatrexate | N-[4-[2-(2,4-Diaminopteridin-6-yl)-1-(2-propynyl)ethyl]benzoyl]-L-glutamic acid 10-Propargyl-10-deazaaminopterin |
| 4 | 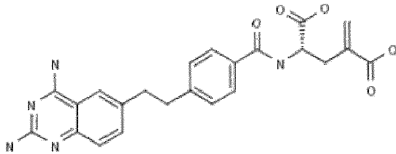 | CH-4051 | N-[4-[2-(2,4-Diaminoquinazolin-6-yl)ethyl]benzoyl]-4-methylene-L-glutamic acid |
| 5 | 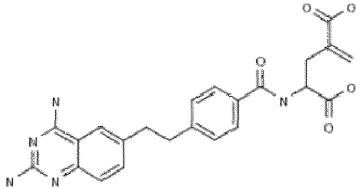 | CH-1504 | N-[4-[2-(2,4-Diaminoquinazolin-6-yl)ethyl]benzoyl]-4-methylene-DL-glutamic acid |
| 6 | 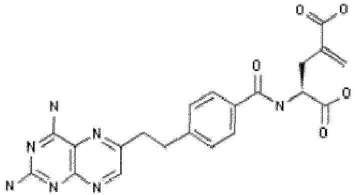 | L-MDAM | N-[4-[2-(2,4-Diaminopteridin-6-yl)ethyl]benzoyl]-4-methylene-L-glutamic acid L-gamma-Methylene-10-deazaaminopterin |
| 7 | 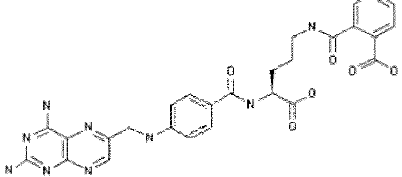 | Talotrexin | Nalpha-(4-Amino-4-deoxypteroyl)-Ndelta-hemiphthaloyl-L-ornithine |
| 8 | 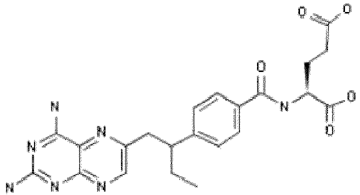 | Edatrexate | 10-Ethyl-10-deazaaminopterin N-[4-[1-(2,4-Diamino-6-pteridinylmethyl)propyl]benzoyl] -L-glutamic acid |
| 9 | 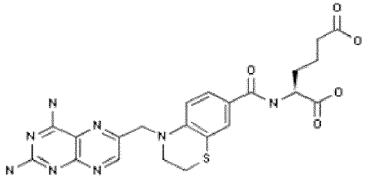 | MX-68 | N-[4-(2,4-Diaminopteridin-6-ylmethyl)-3,4-dihydro-2H-1,4-benzothiazin-7-ylcarbonyl]-L-(3'-homo)glutamic acid 2(S)-[4-(2,4-Diaminopteridin-6-ylmethyl)-3,4-dihydro-2H-1,4-benzothiazi n-7-ylcarboxamido]adipic acid |
| 10 | 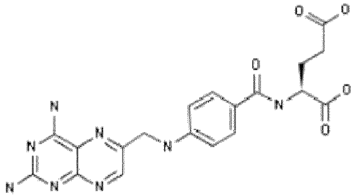 | Aminopterin | N-[4-(2,4-diamino-6-pteridinylmethylamino)benzolyl]-L-glutamic acid |
| 11 | 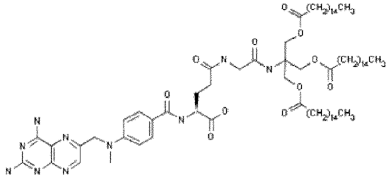 | MTX-gamma-GTP-3 | Nalpha-[4-[N-(2,4-Diamino-6-pteridinylmethyl)-N-methylamino]benzoyl]-Ndelta-[2-[2-(hexadecanoyloxy)-1,1-bis(hexadecanoyloxymethyl)ethyl amino]-2-oxoethyl]-L-glutamine |
| 12 | 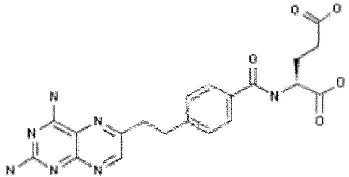 | 10-Deazaaminopteri n | N-[4-[2-(2,4-Diaminopteridin-6-yl)ethyl]benzoyl]-L-glutamic acid |
| 13 | 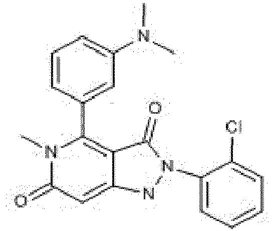 | Setanaxib | 2-(2-Chlorophenyl)-4-[3-(dimethylamino)phenyl]-5-methyl-1H-pyrazolo[4,3-c]pyridine-3,6(2H,5H)-dione |
| 14 | 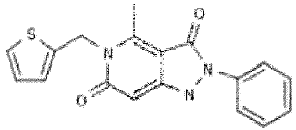 | 640486 | 4-Methyl-2-phenyl-5-(thien-2-ylmethyl)-2,3,5,6-tetrahydro-1H-pyrazolo[4,3-c]pyridine-3,6-dione |
| 15 | 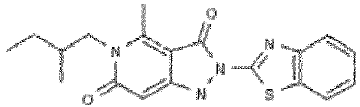 | 640491 | 2-(1,3-Benzothiazol-2-yl)-4-methyl-5-(2-methylbutyl)-2,3,5,6-tetrahydro-1H-pyrazolo[4,3-c]pyridine-3,6-dione |
| 16 | 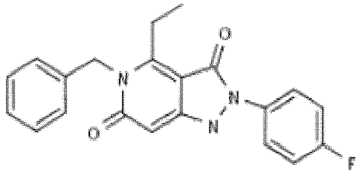 | 640493 | 5-Benzyl-4-ethyl-2-(4-fluorophenyl)-2,3,5,6-tetrahydro-1H-pyrazolo[4,3-c]pyridine-3,6-dione |
| 17 | 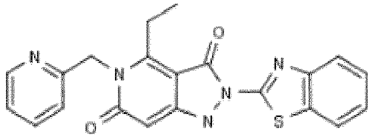 | 640497 | 2-(1,3-Benzothiazol-2-yl)-4-ethyl-5-(pyridin-2-ylmethyl)-2,3,5,6-tetrahydro-1H-pyrazolo[4,3-c]pyridine-3,6-dione |
| 18 | 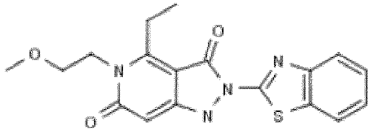 | 640498 | 2-(1,3-Benzothiazol-2-yl)-4-ethyl-5-(2-methoxyethyl)-2,3,5,6-tetrahydro-1H-pyrazolo [4,3-c]pyridine-3,6-dione |
| 19 | 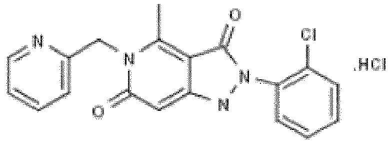 | GKT-136901 | 2-(2-Chlorophenyl)-4-methyl-5-(pyridin-2-ylmethyl)-2,3,5,6-tetrahydro-1H-pyrazolo[4,3-c]pyridine-3,6-dione hydrochloride |
| 20 | 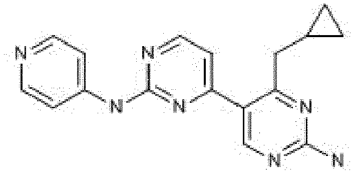 | PIK-III | 4'-(Cyclopropylmethyl)-N2-(pyridin-4-yl)-4,5'-bipyrimidine-2,2'-diamine |
| 21 | 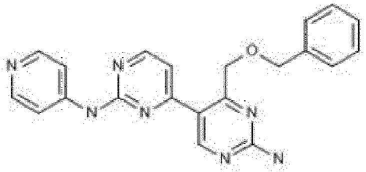 | 781398 | 4'-[(Benzyloxy)methyl]-N2-(pyridin-4-yl)-4,5'-bipyrimidine-2,2'-diamine |
| 22 | 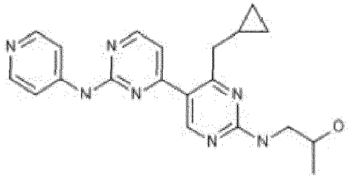 | 781406 | 1-[[4'-(Cyclopropylmethyl)-2-(pyridin-4-ylamino)-4,5'-bipyrimidin-2'-yl]amino]propan-2-ol |
| 23 | 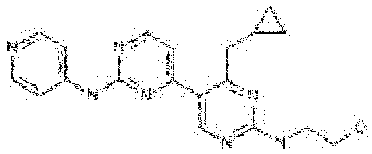 | 781408 | 2-[[4'-(Cyclopropyl methyl)-2-(pyridin-4-ylamino)-4,5'-bipyrimidin-2'-yl]amino]ethanol |
| 24 | 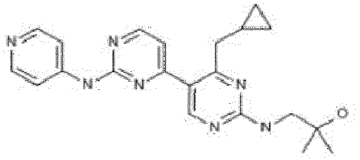 | 781410 | 1-[[4'-(Cyclopropylmethyl)-2-(pyridin-4-ylamino)-4,5'-bipyrimidin-2'-yl]amino]-2-methylpropan-2-ol |
| 25 | 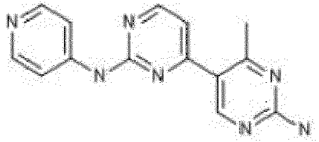 | 781424 | 4'-Methyl-N2-(pyridin-4-yl)-4,5'-bipyrimidine-2,2'-diamine |
| 26 | 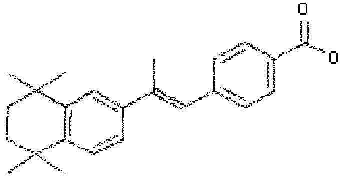 | Arotinoid acid | 4-[2-(5,5,8,8-Tetramethyl-5,6,7,8-tetrahydronaphthalen-2-yl)-1(E)-propenyl]benzoic acid |
| 27 | 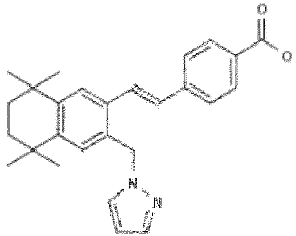 | Palovarotene | 4-[(E)-2-[5,5,8,8-Tetramethyl-3-(1H-pyrazol-1-ylmethyl)-5,6,7,8-tetrahydronaphthalen-2-yl]vinyl]benzoic acid |
| 28 | 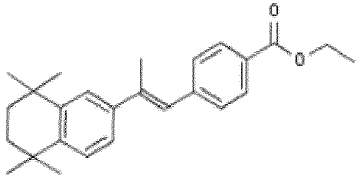 | Ro-136298 | p-[(E)-2-(5,6,7,8-Tetrahydro-5,5,8,8-tetramethyl-2-naphthyl)propenyl]benzoic acid ethyl ester (E)-4-[2-(5,6,7,8-Tetrahydro-5,5,8,8-tetramethyl-2-naphthalenyl)-1-propenyl]benzoic acid ethyl ester |
| 29 | 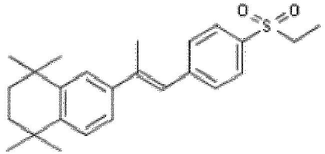 | Etarotene | Ethyl 4-[2(E)-(5,5,8,8-Tetramethyl-5,6,7,8-tetrahydro-2-naphthyl)propenyl]phenylsulfone |
| 30 | 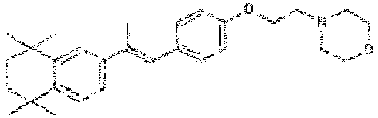 | Mofarotene | 4-[2-[4-[2(E)-(5,5,8,8-Tetramethyl-5,6,7,8-tetrahydro-2-naphthyl)-1-propenyl]phenoxy]ethyl]morpholine |
| 32 | 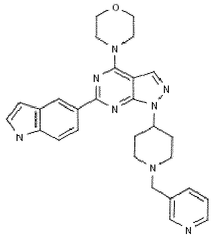 | WAY-600 | 6-(1H-Indol-5-yl)-4-(4-morpholinyl)-1-[1-(pyridin-3-ylmethyl)piperidin-4-yl]-1H-pyrazolo[3,4-d]pyrimidine |
| 33 | 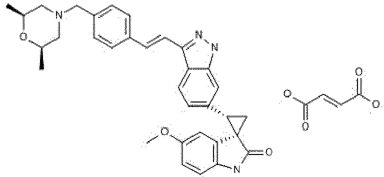 | CFI-400945 | (1R,2S)-2-[3-[(E)-2-[4-[(cis-2,6-Dimethylmorpholin-4-yl)methyl]phenyl]vinyl]-1H-indazol-6-yl]-5'-methoxyspiro[cyclopropane-1,3'-indol]-2'(1'H)-one (2E)-but-2-enedioate |
| 34 | 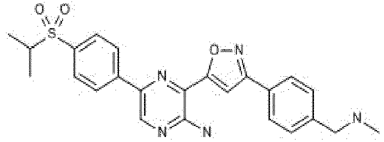 | VE-822 (Berzosertib) | 3-[3-[4-[(Methylamino)methyl]phenyl]-1,2-oxazol-5-yl]-5-[4-(propan-2-ylsulfonyl)phenyl]pyrazin-2-amine |
| 35 | 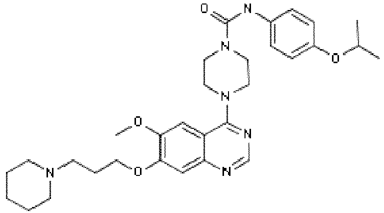 | Tandutinib | N-(4-Isopropoxyphenyl)-4-[6-methoxy-7-[3-(1-piperidinyl)propoxy]quinazolin-4-yl]piperazine-1-carboxamide |
| 36 | 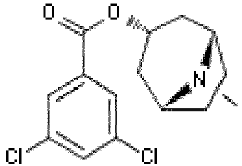 | Bemesetron | 1alphaH,3alpha,5alphaH-Tropan-3-yl-3,5-dichlorobenzoate; 3,5-Dichlorobenzoic acid endo-8-methyl-8-azabicyclo[3.2.1]oct-3-yl ester |
| 37 | 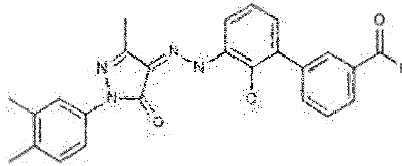 | Eltrombopag | 3'-[2(Z)-[1-(3,4-Dimethyl phenyl)-3-methyl-5-oxo-4,5-dihydro-1H-pyrazol-4-ylidene]hydrazino]-2'-hydroxybiphenyl-3-carboxylic acid |
| 39 | 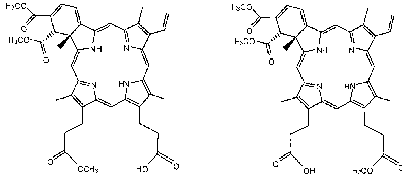 | Verteporfin | (4R,4aS)-18-Ethenyl-4,4a-dihydro-3,4-bis(methoxycarbonyl)-4a,8,14,19-tetramethyl-24H,26H-benzo[b]porphine-9,13-dipropanoic acid monomethyl ester |
| 41 | 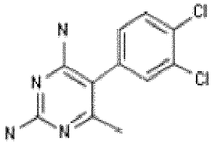 | Metoprine | 5-(3,4-Dichlorophenyl)-6-methylpyrimidine-2,4-diamine |
| 42 | 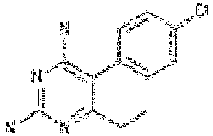 | Pyrimethamine | 5-(4-Chlorophenyl)-6-ethyl-2,4-pyrimidinediamine |
| 43 | 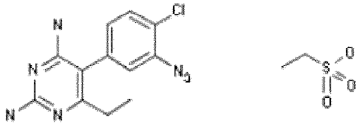 | MZPES | 5-(3-Azido-4-chlorophenyl)-6-ethyl-2,4-pyrimidine-diamine monoethanesulfonate |
| 44 | 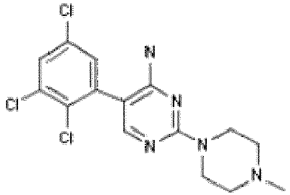 | Sipatrigine | 4-Amino-2-(4-methylpiperazin-1-yl)-5-(2,3,5-trichlorophenyl)pyrimidine 2-(4-Methylpiperazin-1-yl)-5-(2,3,5-trichlorophenyl)pyrimidine-4-amine |
| 45 | 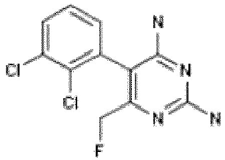 | BW-4030W92 | 5-(2,3-Dichlorophenyl)-6-(fluoromethyl)pyrimidine-2,4-diamine |
| 47 | 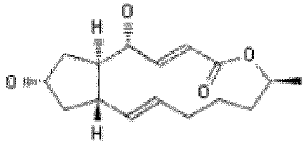 | brefeldin-a | (1R,2S,7S,13S,15S)-2,15-Dihydroxy-7-methyl-6-oxabicyclo[11.3.0]hexadeca-3(E),11(E)-dien-5-one [1S-(1R*,2E,6R*,10E,11aR*,13R*,14a S*)]-1,6,7,8,9,11a,12,13,14,14a-Decahydro-1,13-dihydroxy-6-methyl-4H-cyclopent[f]oxacyclotridecin-4-one |
| 48 | 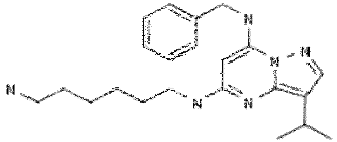 | BS-181 | N5-(6-Aminohexyl)-N7-benzyl-3-isopropylpyrazolo[1,5-a]pyrimidine-5,7-diamine |
| 51 | 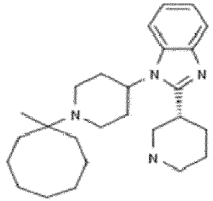 | MCOPPB | 1-[1-(1-Methylcyclooctyl)piperidin-4-yl]-2-[3(R)-piperidinyl]-1H-benzimidazole |
| 52 | 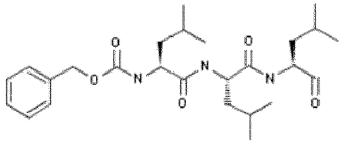 | MG-132 | Benzyloxycarbonyl-L-leucyl-L-leucyl-L-leucinal |
| 53 | 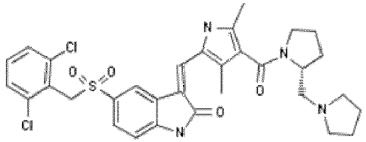 | PHA-665752 | 5-(2,6-Dichlorobenzylsulfonyl)-3(Z)-[3,5-dimethyl-4-[2(R)-(pyrrolidin-1-ylmethyl)pyrrolidin-1-ylcarbonyl]-1H-pyrrol-2-ylmethylene]-2,3-dihydro-1H-indol-2-one |
| 54 | 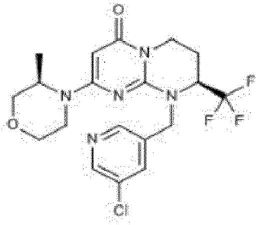 | SAR405 | (8S)-9-[(5-Chloropyridin-3-yl)methyl]-2-[(3R)-3-methylmorpholin-4-yl]-8-(trifluoromethyl)-6,7,8,9-tetrahydro-4H-pyrimido[1,2-a]pyrimidin-4-one |
| 56 | 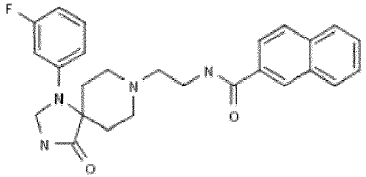 | VU-0364739 | N-[2-[1-(3-Fluorophenyl)-4-oxo-1,3,8-triazaspiro[4.5]dec-8-yl]ethyl]naphthalen-2-carboxamide |
| 57 | 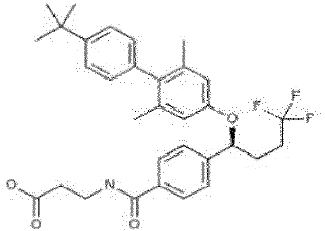 | ADOMEGLIVANT | N-(4-[(1S)-1-[(4'-tert-Butyl-2,6-dimethylbiphenyl-4-yl)oxy]-4,4,4-trifluorobutyl]benzoyl)-beta-alanine |
| 59 | 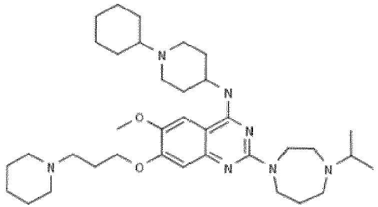 | UNC0646 | N-(1-Cyclohexylpiperidin-4-yl)-6-methoxy-7-[3-(1-piperidinyl)propoxy]-2-[4-(propan-2-yl)perhydro-1,4-diazepin-1-yl]quinazolin-4-amine |
| 60 | 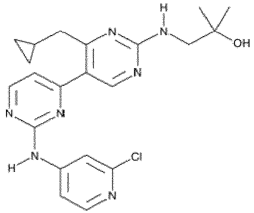 | VPS34-IN-1 | 1-[[2-[(2-chloro-4-pyridinyl)amino]-4'-(cyclopropyl methyl) [4,5'-bipyrimidin]-2'-yl]amino]-2-methyl-2-propanol |
| 61 | 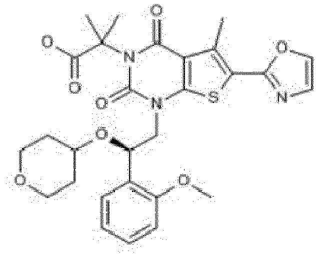 | ND-630 | 2-[1-[(2R)-2-(2-Methoxyphenyl)-2-(tetrahydro-2H-pyran-4-yloxy)ethyl]-5-methyl-6-(1,3-oxazol-2-yl)-2,4-dioxo-1,4-dihydrothieno[2,3-d]pyrimidin-3(2H)-yl]-2-methylpropanoic acid |
| 62 | 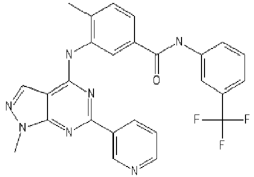 | NVP-BHG712 | 4-methyl-3-{[1-methyl-6-(3-pyridinyl)-1H-pyrazolo[3,4-d]pyrimidin-4-yl]amino}-N-[3-(trifluoromethyl)phenyl]benzami de |
| 63 | 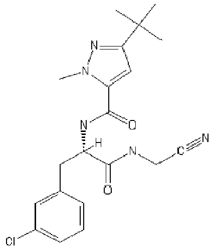 | Cathepsin Inhibitor 1 | 3-tert-butyl-N-{(1S)-1-(3-chlorobenzyl)-2-[(cyanomethyl)amino]-2-oxoethyl}-1-methyl-1H-pyrazole-5-carboxamide |
| 64 | 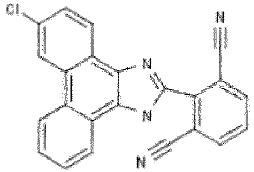 | MF63 | 2-(6-Chloro-1H-phenanthro[9,10-d]imidazol-2-yl)benzene-1,3-dicarbonitrile |
| 65 | 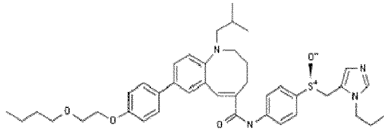 | CENICRIVIROC | (-)-(S)-8-[4-(2-Butoxyethoxy)phenyl]-1-isobutyl-N-[4-(1-propyl-1H-imidazol-5-ylmethylsulfinyl)phenyl]-1,2,3,4-tetrahydro-1-benzazocine-5-carboxamide |
| 66 | 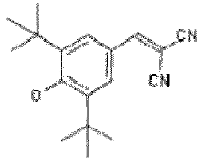 | TYRPHOSTIN A9 | (3,5-Di-tert-butyl-4-hydroxybenzylidene)malononitril e |
| 67 | 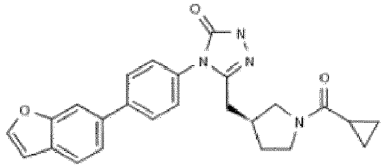 | GSK2194069 | 4-[4-(1-Benzofuran-6-yl)phenyl]-5-[1-(cyclopropylcarbonyl)pyrrolidin-3(S)-ylmethyl]-3,4-dihydro-2H-1,2,4-triazol-3-one |
| 69 | 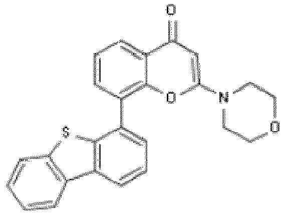 | NU 7441 | 8-(Dibenzo[b,d]thien-4-yl)-2-(4-morpholinyl)-4H-1-benzopyran-4-one |
| 70 | 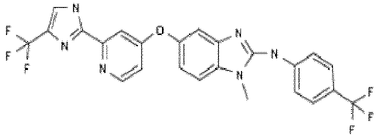 | CHIR-265 | 1-Methyl-N-[4-(trifluoromethyl)phenyl]-5-[2-[4-(trifluoromethyl)-1H-imidazol-2-yl]pyridin-4-yloxy]-1H-benzimidazol-2-amine |
| 72 | 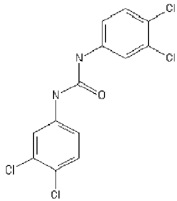 | MMV665852 | 1,3-Bis(3,4-dichlorophenyl)urea |
| 73 | 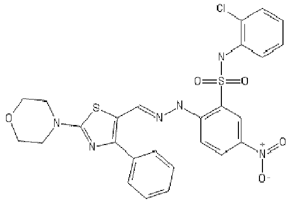 | PB49673382 | N-(2-chlorophenyl)-2-[(2E)-2-[(2-morpholin-4-yl-4-phenyl-1,3-thiazol-5-yl)methylidene]hydrazinyl]-5-nitrobenzenesulfonamide |
| 74 | 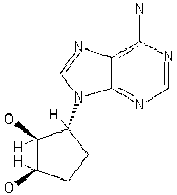 | DHCaA | (1R,2S,3R)-9-(2,3-Dihydroxycyclopentyl)adenine (1R,2S,3R)-3-(6-Amino-9H-purin-9-yl)-1,2-cyclopentanediol |
| 75 | 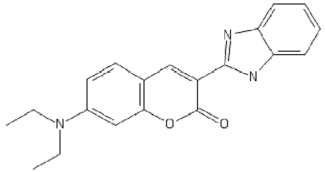 | Coumarin 7 | (1R,2S,3R)-3-(6-Amino-9H-purin-9-yl)-1,2-cyclopentanediol |
| 76 | 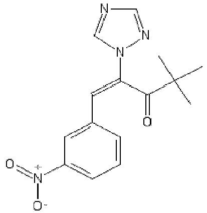 | Nexinhib20 | 4,4-dimethyl-1-(3-nitrophenyl)-2-(1H-1,2,4-triazol-1-yl)-1-penten-3-one |
| 78 | 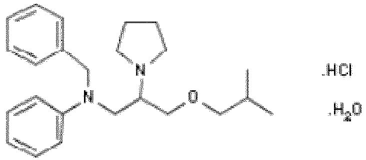 | Bepridil | 1-[1-(N-Benzylanilino)-3-isobutoxy-2-propyl]pyrrolidine monohydrochloride monohydrate |
| 79 | 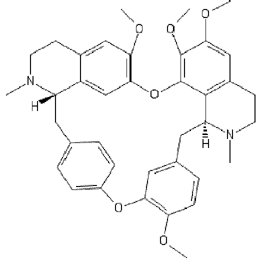 | Tetrandrine (Fanchinine) | (1beta)-(S,S)-6,6',7,12-Tetramethoxy-2,2'-dimethylberbaman |
| 80 | 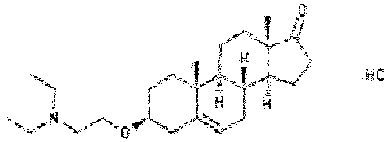 | U-18666° | 3beta-[2-(Diethylamino)ethoxy]androst-5-en-17-one hydrochloride |
| 81 | 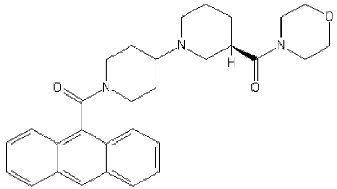 | CP-640186 | 1-[1'-(Anthracen-9-ylcarbonyl)-1,4'-bipiperidin-3(R)-yl]-1-(4-morpholinyl)methanone |
| 82 | 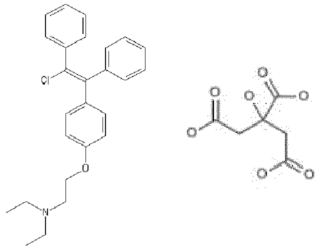 | CLOMIPHENE CITRATE | 2-[4-(2-Chloro-1,2-diphenylvinyl)phenoxy]-N,N-diethylethanamine citrate |
| 84 | 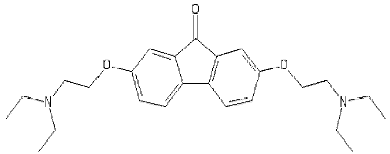 | TILORONE | 2,7-Bis[2-(diethylamino)ethoxy]-9H-fluoren-9-one |
| 85 | 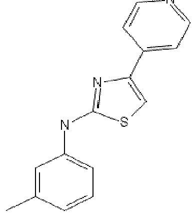 | STF-62247 | N-(3-Methylphenyl)-4-(4-pyridyl)thiazol-2-amine |
| 86 | 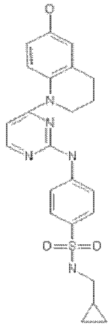 | Pyrintegrin | N-(Cyclopropylmethyl)-4-[[4-(3,4-dihydro-6-hydroxy-1(2H)-quinolinyl)-2-pyrimidinyl]amino]benzenesulfo namide |
| 87 | 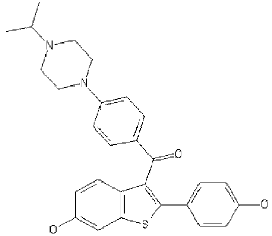 | Y-134 | 1-[6-Hydroxy-2-(4-hydroxyphenyl)-1-benzothien-3-yl]-1-[4-(4-isopropylpiperazin-1-yl)phenyl]methanone |
| 89 | 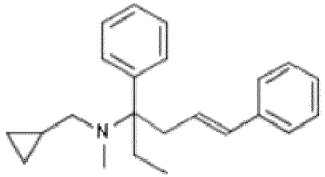 | Igmesine | (+)-N-(Cyclopropylmethyl)-N-[1-ethyl-1,4-diphenyl-3(E)-butenyl]-N-methylamine |
| 90 | 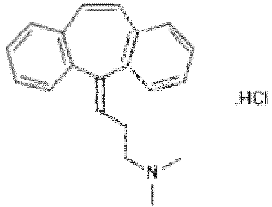 | Cyclobenzaprine Hydrochloride | 3-(5H-Dibenzo[a,d]cyclohepten-5-ylidene)-N,N-dimethylpropan-1-amine hydrochloride |
| 92 | 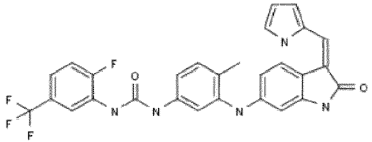 | GNF-5837 | 1-[2-Fluoro-5-(trifluoromethyl)phenyl]-3-[4-methyl-3-[2-oxo-3-(1H-pyrrol-2-ylmethylene)-2,3-dihydro-1H-indol-6-ylamino]phenyl]urea |
| 94 | 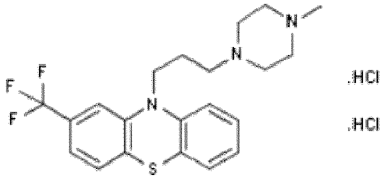 | Trifluoperazine dihydrochloride | 10-[3-(4-Methylpiperazin-1-yl)propyl]-2-(trifluoromethyl)-10H-phenothiazine dihydrochloride |
| 95 | 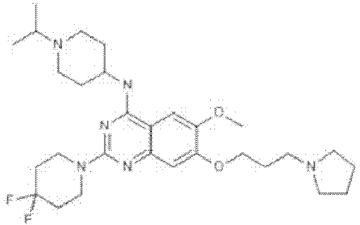 | UNC0642 | 2-(4,4-Difluoropiperidin-1-yl)-6-methoxy-N-[1-(propan-2-yl)piperidin-4-yl]-7-[3-(pyrrolidin-1-yl)propoxy]quinazolin-4-amine |
| 98 | 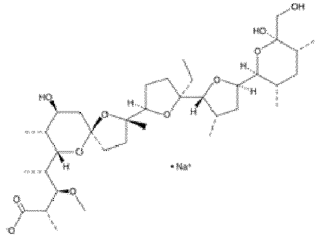 | Monensin Sodium | 4(S)-[(2S,5R,7S,8R,9S)-2-[(2S,2'R,3'S,5R,5'R)-2-Ethyl-5'-[6(R)-hydroxy-3(S),5(R)-dimethyl-6-(phenylaminocarbonyloxymethyl )tetrahydropyran-2(S)-yl]-3'-methylperhydro-2,2'-bifuran-5-yl]-9-hydroxy-2,8-dimethyl-1,6-dioxaspiro[4.5]dec-7-yl]-3(R)-methoxy-2(S)-methylpentanoic acid sodium salt |
| 99 | 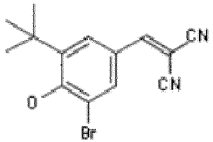 | AG1024 | 2-(3-Bromo-5-tert-butyl-4-hydroxybenzylidene)propanedini trile 2-(3-Bromo-5-tert-butyl-4-hydroxybenzylidene)malononitrile |
| 101 | 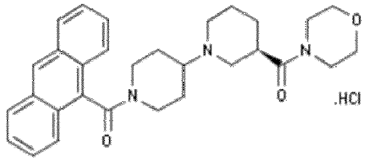 | CP-640186 hydrochloride | 1-[1'-(Anthracen-9-ylcarbonyl)-1,4'-bipiperidin-3(R)-yl]-1-(4-morpholinyl)methanone hydrochloride |
| 102 | 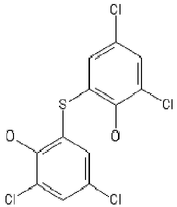 | Bithionol | 2,2'-Thio-bis(4,6-dichlorophenol), Bis(2-hydroxy-3,5-dichlorophenyl) sulfide |
| 103 | 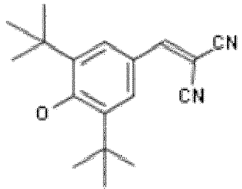 | Tyrphostin 9 | (3,5-Di-tert-butyl-4-hydroxybenzylidene)malononitrile |
| 104 | 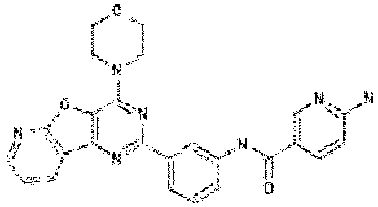 | YM201636 | 6-Amino-N-[3-[4-(4-morpholinyl)pyrido[3',2':4,5]furo [3,2-d]pyrimidin-2-yl]phenyl]pyridine-3-carboxamide |
| 105 | 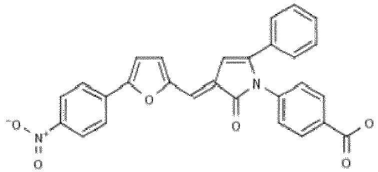 | 4E1RCat | 4-[3-[5-(4-Nitrophenyl)furan-2-ylmethylene]-2-oxo-5-phenyl-2,3-dihydro-1H-pyrrol-1-yl]benzoic acid |
| 108 | 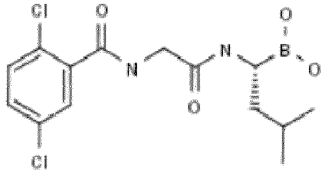 | Ixazomib | 1(R)-[2-(2,5-Dichlorobenzamido)acetamido]-3-methylbutylboronic acid |
| 109 | 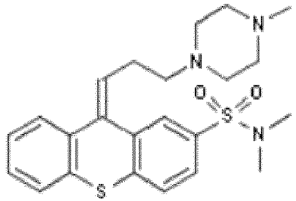 | Thiothixene | (Z)-N,N-Dimethyl-9-[3-(4-methyl-1-piperazinyl)propylidene]thioxant hene-2-sulfonamide |

The term "salt" of a compound is understood by the skilled person. The salt may be formed by conventional means, such as e.g. by reacting the free form of the compound with one or more equivalents of the appropriate acid or base in a solvent or medium in which the salt is insoluble, such as for example water or ethanol, which is then preferably removed, e.g. under vacuum or by freeze drying. Preferably, the salt is a pharmaceutically acceptable salt. As used herein the term "pharmaceutically acceptable salts" of the compounds of Table 1 above refers to addition salts of these compounds with acids or bases that form anions or cations, which are, preferably, non-toxic to the recipient thereof. Examples of acid addition salts include acetate, adipate, ascorbate, benzoate, benzenesulfonate, bicarbonate, bisulfate, butyrate, camphorate, camphorsulfonate, choline, citrate, cyclohexyl sulfamate, diethylenediamine, ethanesulfonate, fumarate, glutamate, glycolate, hemisulfate, 2-hydroxyethylsulfonate, heptanoate, hexanoate, hydrochloride, hydrobromide, hydroiodide, hydroxymaleate, lactate, malate, maleate, methanesulfonate, meglumine, 2-naphthalenesulfonate, nitrate, oxalate, pamoate, persulfate, phenylacetate, phosphate, diphosphate, picrate, pivalate, propionate, quinate, salicylate, stearate, succinate, sulfamate, sulfanilate, sulfate, tartrate, tosylate (p-toluenesulfonate), trifluoroacetate, and undecanoate salts. Examples of base addition salts include ammonium salts; alkali metal salts such as sodium, lithium and potassium salts; alkaline earth metal salts such as aluminum, calcium and magnesium salts; salts with organic bases such as dicyclohexylamine salts and N-methyl-D-glucamine; and salts with amino acids such as arginine, lysine, ornithine, and so forth. Also, basic nitrogen-containing groups may be quaternized with such agents as: lower alkyl halides, such as methyl, ethyl, propyl, and butyl halides; dialkyl sulfates such as dimethyl, diethyl, dibutyl; diamyl sulfates; long chain halides such as decyl, lauryl, myristyl and stearyl halides; arylalkyl halides such as benzyl bromide and others.

The compound may preferably also be a prodrug of a compound indicated in Table 1 or of a salt thereof. As used herein, the term "prodrug" refers to a biologically inactive or essentially inactive compound which can be metabolized in the body to produce a compound of Table 1 above. Thus, preferably, a prodrug is an ether or preferably an ester of a compound. More preferably, the prodrug is a glycosylate, a phosphate, a sulphate, or a macromolecule-conjugated, e.g. polyethyleneglycol (PEG) conjugated, derivative of the compound. Preferably, the prodrug is a compound facilitating its transmittal across at least one cell membrane. In an example, without limitation, a prodrug is an ester of compound of Table 1 or salt thereof, that facilitates transmittal across a cell membrane where water solubility is not beneficial, but then is metabolically hydrolysed once inside the cell where water solubility is beneficial.

Preferably, the compound is comprised in a composition. The term "composition", as used herein, relates to a composition of matter comprising the compound as specified and optionally one or more acceptable carrier(s) and/or further pharmaceutically active compound. Preferably, the composition is a non-naturally occurring composition. Preferably, the composition is a pharmaceutic composition; thus, the composition, preferably, comprises the compound(s) as specified as pharmaceutically active compound, and, preferably, the carrier is a pharmaceutically acceptable carrier. The pharmaceutically active compound(s) can be formulated as, preferably pharmaceutically acceptable, salt(s), preferably as specified herein above. The composition comprises at least one compound as indicated; thus, the composition preferably comprises one compound as specified or comprises a multitude of compounds, the term "multitude", preferably referring to at least two. Thus, the composition may comprise a multitude of compounds selected from Table 1, e.g. at least two or at least three compounds of Table 1. Preferably, the composition is a pharmaceutical composition, i.e., preferably, a medicament.

The terms "medicament" and "pharmaceutical composition" are, in principle, known to the skilled person. As referred to herein, the term relate to any composition containing a compound of Table 1 or salt or prodrug thereof as pharmaceutically active compound and one or more other components such as one or more pharmaceutically acceptable excipient(s), including e.g. pharmaceutically acceptable carrier(s), the term "pharmaceutically acceptable excipient" preferably including any and all material suitable for preparing a pharmaceutical composition to be administered to a living being without being deleterious thereto. Thus, the term includes any solvents, diluents, or other vehicle, dispersion or suspension aids, surface active agents, isotonic agents, thickening or emulsifying agents, preservatives, solid binders, lubricants and the like, as suited to the particular dosage form desired. Some examples of materials which can serve as pharmaceutically acceptable excipient include, but are not limited to, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatine; talc; excipients such as cocoa butter and suppository waxes; oils such as peanut oil, cottonseed oil; safflower oil; sesame oil; olive oil; corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; sterilized water; Ringer's solution; buffered saline; dextrose solution; maltodextrin solution; ethyl alcohol; and phosphate buffer solutions. The pharmaceutically active compound preferably is present in the medicament in liquid or dry, e.g. lyophilized, form. The pharmaceutically active compound is the active ingredient or drug of the medicament, and is preferably administered in a conventional dosage form prepared by combining the drug with standard pharmaceutical excipients according to conventional procedures. These procedures may involve mixing, granulating, compression, and/or dissolving the ingredients as appropriate to the desired preparation. It will be appreciated that the form and character of the pharmaceutical acceptable carrier or diluent is dictated by the amount of active ingredient with which it is to be combined, the route of administration, and other well-known variables. The excipient(s) must be acceptable in the sense of being compatible with the other ingredients of the formulation and being not deleterious to the recipient thereof. In addition, the pharmaceutical composition or formulation may also include other carriers, adjuvants, preservatives, stabilizers, surfactants, buffers, and/or salts for regulating osmotic pressure, emulsifiers, sweeteners, colorants, flavourings and the like. Preferably, the amount of the compound in the pharmaceutical composition is the the amount usually employed for other indications of the specific compound. The amount can vary over a wide range depending on known factors, for example, as specified herein below. However, a person skilled in the art can determine the optimum amount in easy and routine manner, in particular based on the existing dosage recommendations for the compound.

The route of administration of the compound or pharmaceutical composition of the present invention depends on the specific compound used or contained in the pharmaceutical composition and is in accordance with known methods for administration, which can be, for example inhalation, injection or infusion by intravenous, parenterally, topically, rectally, nasally, buccally, vaginally or via an implanted reservoir, or by sustained release systems. The term parenteral as used herein includes intravenous, intraperitoneal, intracerebral, intrathecal, intracranial, intramuscular, intraarticular, intrasynovial, intrasternal, intraocular, intraarterial, subcutaneous, and intracutaneous or intralesional injection or infusion techniques. Preferably, the compound or the pharmaceutical composition is administered such that a concentration sufficient to achieve the effect of treating and/or preventing coronavirus infection is achieved, i.e. such that an effective concentration is achieved. Moreover, the composition may be formulated into inhalable or injectable dosage forms such as solutions, suspensions, and emulsions by further adding diluents, dispersants, and surfactants. Further, the composition may be suitably formulated using appropriate methods known in the art or by the method disclosed in Remington's Pharmaceutical Science (recent edition), Mack Publishing Company, Easton Pa. Preferably, the compound or the pharmaceutical composition is administered such that an effective concentration is reached in the respiratory tract. Preferably, this is obtained by direct administration of the compound or pharmaceutical composition to the respiratory tract. The direct administration to the respiratory tract may be the sole route of administration of the compound or composition or may be combined with administration of the compound or composition via other, preferably systemic, routes. For direct administration of the compound or composition, a wide variety of devices that are designed for the direct delivery of pharmaceutical compositions and therapeutic formulations in particular to the respiratory tract, may be used. Preferably, the compound or pharmaceutical composition is administered in aerosolized or inhaled form; however, administration via a lavage may also be envisaged. The pharmaceutical composition for direct administration to the respiratory tract as described above, can be in form of an aerosol formulation, as a dry powder or a solution or suspension with a diluent.

The pharmaceutical composition is, preferably, administered in conventional dosage forms prepared by combining the active compound with standard pharmaceutically acceptable excipients and/or carriers as specified herein above and according to conventional procedures. A therapeutically effective dose refers to an amount of the active compound to be used in a pharmaceutical composition which provides the effect referred to herein. Therapeutic efficacy and toxicity of compounds can be determined by standard pharmaceutical procedures in cell cultures or experimental animals, e.g., ED50 (the dose therapeutically effective in 50% of the population) and LD50 (the dose lethal to 50% of the population), or IC50 (the dose causing 50% inhibition). The dose ratio between therapeutic and toxic effects is the therapeutic index, and it can be expressed as the ratio, LD50/ED50. The dosage regimen will be determined by the attending physician and other clinical factors; preferably in accordance with any one of the above described methods. As is well known in the medical arts, dosages for any one patient depend upon many factors, which may include the patient's size, body surface area, age, the particular compound to be administered, sex, time and route of administration, general health, and other drugs being administered concurrently. Progress can be monitored by periodic assessment. A typical dose can be, for example, in the range of 1 µg to 1000 mg; however, doses below or above this exemplary range are envisioned, especially considering the aforementioned factors. Generally, the regimen as a regular administration of the pharmaceutical composition should be in the range of 1 µg to 100 mg units per day. If the regimen is a continuous infusion, it should also be in the range of 1 µg to 1 mg units per kilogram of body weight per minute, respectively. Preferably, the pharmaceutical composition is administered once to the subject, i.e., preferably, is used as a one-time treatment. Depending on the subject and the mode of administration, the quantity of substance administration may vary over a wide range to provide from about 0.01 mg per kg body mass to about 100 mg per kg body mass. The pharmaceutical compositions and formulations referred to herein are administered at least once in order to treat or ameliorate or prevent a disease or condition recited in this specification. However, the said pharmaceutical compositions may be administered more than one time, for example from two to 50 times, more preferably from five to 50 times. Administration may be continuous, e.g. as an infusion or by inhalation, hourly, two to 6 times daily, daily, every other day, every three days, weekly, biweekly, or monthly, preferably is continuous, hourly, two to 6 times daily, or daily, Preferably, administration is adjusted to maintain an effective concentration in the body of a subject for the time period intended. Progress can be monitored by periodic assessment. Preferably, the dosage of the compound is in accordance with the known indications of said compound; however, other dosages, in particular higher doses, may be envisaged.

The term "treating", as used herein, refers to ameliorating or curing a disease or at least one symptom associated therewith. Thus, if there is amelioration or cure of the disease or at least a symptom associated therewith, the treatment shall be deemed to be effective. It will be understood that treating might not be effective in all subjects. However, it is envisaged that treatment will preferably be effective in at least a statistically significant portion of subjects to be treated. It is well known to the skilled artisan how to determine a statistically significant portion of subjects that can be effectively treated. Whether a portion is statistically significant can be determined without further ado by the person skilled in the art using various well known statistic evaluation tools, e.g., determination of confidence intervals, p-value determination, Student's t-test, Mann-Whitney test etc. Details are found in Dowdy and Wearden, Statistics for Research, John Wiley & Sons, New York 1983. Preferred confidence intervals are at least 90%, at least 95%, at least 97%, at least 98% or at least 99%. The p-values are, preferably, 0.1, 0.05, 0.01, 0.005, or 0.0001. Preferably, the probability envisaged by the present invention allows that the finding of coronavirus infection will be correct for at least 60%, at least 70%, at least 80%, or at least 90% of the subjects of a given cohort or population. Preferably, treating is treating of a subject known to be infected with a coronavirus as specified herein and/or showing at least one symptom of coronavirus infection, preferably at least one of hypoxia, fever, cough, in particular dry cough, pneumonia, acute respiratory distress syndrome, sepsis, septic shock, and kidney failure. More preferably, treating is treating of a subject known to be infected with a coronavirus as specified herein and showing at least one symptom of coronavirus infection, preferably at least one of hypoxia, fever, cough, in particular dry cough, pneumonia, acute respiratory distress syndrome, sepsis, septic shock, and kidney failure. Even more preferably, treating is treating of a subject known to be infected with a coronavirus as specified herein and requiring emergency care.

The term "preventing" as used herein refers to avoiding the onset of the disease or at least one symptom associated therewith or to prevent the worsening of the disease or the said at least one symptom. The prevention as referred to herein can be typically achieved shortly after the compound is administered. If the administration stopped, however, the prevention may not persist for an unlimited time but may remain present for a certain preventive time window after application of the drug. Typically, a preventive time window in accordance with the present invention may be at least 1 day, at least 2 days, at least 3 days, at least 4 days, at least 5 days, at least 7 days, or at least two or four weeks. However, the preventive time window may also depend on the dosage of the compound(s) as well as the mode of administration, the kind of formulation, and/or the number of administrations. For example, if a high dosage is applied, usually longer preventive time windows can be achieved. The same holds true if repeated doses are administered. It will be understood that prevention might not be effective in all subjects. However, according to the present invention it is envisaged that prevention preferably will be effective in at least a statistically significant portion of subjects. It is well known to the skilled artisan how to determine a statistically significant portion of subjects that can be effectively prevented. Whether a portion is statistically significant can be determined without further ado by the person skilled in the art using various well known statistic evaluation tools as discussed above. Preferably prevention is general prevention of coronavirus infection, of spread of coronavirus infection, or of onset of symptoms thereof in a subject, preferably of the general population; more preferably, prevention is prevention of coronavirus infection, of spread of coronavirus infection, or of onset of symptoms thereof in a subject known or suspected to be infected with a coronavirus as specified herein above.

Preferably, treating and/or preventing comprises inhibition of coronavirus replication. Also, preferably, treating and/or preventing comprises inhibition of cell lysis by said coronavirus. Further preferably, treating and/or preventing comprises inhibition of tissue destruction, preferably lung tissue destruction, by said coronavirus. As is understood by the skilled person, the term "inhibition" includes partial inhibition. Thus, in the case of a quantifiable event, inhibition preferably is a reduction of said event by at least 25%, more preferably at least 50%, still more preferably at least 75%, most preferably at least 85%. As will be understood by the skilled person, inhibition may also include complete inhibition, i.e. prevention of an event or process from occurring. Exemplary methods for determining inhibition by the compounds specified herein are provided herein in the Examples.

The term "coronavirus" is understood by the skilled person to relate to a group of enveloped viruses from the order Nidovirales, having a positive-sense single-stranded RNA genome with a size of approx. 25 to 35 kilobases. Preferably, the coronavirus is a beta-coronavirus, more preferably a severe acute respiratory syndrome coronavirus (SARS-CoV)-2, SARS-CoV-1, or Middle East respiratory syndrome coronavirus (MERS-CoV). Preferably the coronavirus is SARS-CoV-2. The terms "SARS-CoV-2" and "severe acute respiratory syndrome coronavirus 2" are understood by the skilled person. Preferably, the terms relate to the virus identified in Genbank entry NCBI:txid2697049 or a strain thereof. Symptoms and diseases caused by coronavirus infection and in particular SARS-CoV-2 infection are known to the skilled person. Also preferably the coronavirus is SARS-CoV-1 or Middle East respiratory syndrome coronavirus (MERS-CoV, NCBI:txid1335626).

The term "subject" as used herein refers to any kind of animal encompassing, e.g., mammals, birds, fish or reptiles. Typically, the animal, however, is a mammal such as a mammals used as pets including dogs, cats, horses, or rodents, laboratory animals, e.g., rats, mice or apes, or farming animals such as pigs, cows, goats, or sheep. More preferably, the mammal is a primate and, most preferably, a human. The subject according to the present invention shall preferably be known or suspected to suffer from coronavirus infection. Preferably, the subject is an animal known or suspected to be infectable by a coronavirus as specified, more preferably is a human. More preferably, in particular in case the compound is for use in treating a coronavirus infection, the subject has been diagnosed to suffer from coronavirus infection. The identification in a subject of a SARS-CoV-2 infection is preferably based on the analysis of the presence of viral RNA in biological material from the subject, preferably specimens from upper and lower respiratory tract, such as nasopharyngeal/oropharyngeal swabs, nasal aspirate, nasal wash or saliva, sputum, tracheal aspirate or bronchoalveolar lavage. Preferably, the subject to be treated may have one or more symptoms of SARS-CoV-2 infection or may be asymptomatic.

Advantageously, the inventors have carried out the analysis of a library containing more than 10.000 commercialized drugs and clinical candidates "safe in man" or characterized up to late clinical stage and have selected by Computer-Aided Drug Design a number of molecules able to bind to SARS-Cov-2 functional proteins essential for the pathogenetic mechanisms. Furthermore, the inventors have confirmed by in vitro screening the ability of the selected molecules to inhibit virus survival and replication in infected host cells. The compounds selected are described in detail herein. Thus, it was found in the work underlying the present invention that the compounds referred to herein are active in inhibiting replication of SARS-CoV-2 in a cell culture system, making the compounds candidates for treatment and prevention of coronavirus infections.

The definitions made above apply mutatis mutandis to the following. Additional definitions and explanations made further below also apply for all embodiments described in this specification mutatis mutandis.

The present invention further relates to a composition comprising a compound selected from the compounds indicated in Table 1, pharmaceutically acceptable salts, and prodrugs thereof for use in treating and/or preventing a coronavirus infection in a subject.

The present invention also relates to a method of treating and/or preventing a coronavirus infection in a subject, comprising administering a compound as specified herein above to said subject, thereby treating and/or preventing said coronavirus infection in said subject.

The method of treating and/or preventing a coronavirus infection preferably, is an in vivo method, preferably performed on a subject's body, preferably on a human body. Moreover, the method may comprise steps in addition to those explicitly mentioned above. For example, further steps may relate, e.g., to administering further treatment as specified herein below before, during, or after administering the compound as specified. Moreover, one or more of said steps may be performed by automated equipment.

Further treatment which may be optionally administered preferably includes administration of further medication, e.g. medication for sepsis treatment, further medication for inhibiting virus replication, immunosuppressive medication, and the like. Further treatment which may be optionally administered also preferably includes administration of oxygen and/or ventilation.

The invention also relates to a method of inhibiting coronavirus replication in a cell or tissue, comprising contacting said cell or tissue with a compound selected from the compounds indicated in Table 1, pharmaceutically acceptable salts, and prodrugs thereof.

The method of inhibiting coronavirus replication in a cell or tissue preferably is an in vitro method. Also the method may comprise steps in addition to those mentioned above, e.g. providing a cell or issue, contacting said cell or tissue with a coronavirus, or analyzing coronavirus replication during or after contacting with the compound of Table 1.

The present invention further relates to a use of a compound selected from the compounds indicated in Table 1, pharmaceutically acceptable salts, and prodrugs thereof for the manufacture of a medicine for treating and/or preventing a coronavirus infection in a subject.

Also, the instant invention relates to a kit comprising a compound or a composition according to the invention comprised in a housing.

The term "kit", as used herein, refers to a collection of the aforementioned compounds, compositions, means, or reagents which may or may not be packaged together. The components of the kit may be comprised by separate vials (i.e. as a kit of separate parts) or provided in a single vial. Moreover, it is to be understood that the kit of the present invention, preferably, is to be used for practicing the methods or uses referred to herein above. It is, preferably, envisaged that all components are provided in a ready-to-use manner for practicing the methods or uses referred to above. Further, the kit, preferably, contains instructions for carrying out said methods or uses. The instructions can be provided by a user's manual in paper or electronic form. In addition, the manual may comprise instructions for administration and/or dosage instructions using the kit of the present invention.

Preferably, the kit comprises a diluent and/or a means of administration. Appropriate diluents are described herein above; means of administration are all means suitable for administering the compound to a subject, a cell, or a tissue. Preferred means of administration are those known to the skilled person for the respective compound. The means of administration may include a delivery unit for the administration of the compound or composition and a storage unit for storing said compound or composition until administration. However, it is also contemplated that the means of the current invention may appear as separate devices in such an embodiment and are, preferably, packaged together in said kit. Preferred means for administration are those which can be applied without the particular knowledge of a specialized technician. In a preferred embodiment, the means for administration is a syringe, more preferably with a needle, comprising the compound or composition of the invention. In another preferred embodiment, the means for administration is an intravenous infusion (IV) equipment comprising the compound or composition. In still another preferred embodiment the means for administration is an inhaler comprising the compound of the present invention, wherein, more preferably, said compound is formulated for administration as an aerosol.

The present invention also relates to a device comprising a compound of the present invention.

The term "device", as used herein, relates to a system of means comprising at least the means operatively linked to each other as to allow administration of the compound or composition, in particular the medicament, of the present invention. Thus, the device preferably is adapted for administration of the compound or composition. Preferred means for administering a compound or composition depend on the mode of administration envisaged and are well known in the art. How to link the means in an operating manner will depend on the type of means included into the device. Preferably, the means are comprised by a single device. The device may include a delivery unit for the administration of the compound or composition and a storage unit for storing said compound or composition until administration. However, it is also contemplated that the means of the current invention may appear as separate devices in such an embodiment and are, preferably, packaged together as a kit. The person skilled in the art will realize how to link the means without further ado. Preferred devices are those which can be applied without the particular knowledge of a specialized technician. Preferably, the device is a syringe, more preferably with a needle, comprising the compound or composition of the invention. Also preferably, the device is an intravenous infusion (IV) equipment comprising the compound or composition. Further preferably, the device is an endoscopic device comprising the compound or composition for flushing the lung or parts thereof or a body cavity. Also preferably, the device is an inhaler comprising the compound or composition, wherein, more preferably, said compound or composition is formulated for administration as an aerosol; more preferably, the device is an oxygen supply device and/or a ventilator in such case.

In view of the above, the following embodiments are particularly envisaged:
Embodiment 1: A compound selected from the compounds indicated in Table 1 and pharmaceutically acceptable salts and prodrugs thereof for use in treating and/or preventing a coronavirus infection in a subject.
Embodiment 2: The compound for use of embodiment 1, wherein said compound is selected from the compounds indicated in Table 1 and pharmaceutically acceptable salts thereof.
Embodiment 3: The compound for use of embodiment 1 or 2, wherein said compound, pharmaceutically acceptable salt, or prodrug is comprised in a composition, preferably a pharmaceutical composition.
Embodiment 4: The compound for use of any one of embodiments 1 to 3, wherein said coronavirus is severe acute respiratory syndrome coronavirus (SARS-CoV)-2, SARS-CoV-1, or Middle East respiratory syndrome coronavirus (MERS-CoV).
Embodiment 5: The compound for use of any one of embodiments 1 to 4, wherein said coronavirus is SARS-CoV-2.
Embodiment 6: The compound for use of any one of embodiments 1 to 5, wherein said treating and/or preventing comprises inhibition of coronavirus replication.
Embodiment 7: The compound for use of any one of embodiments 1 to 6, wherein said treating and/or preventing comprises inhibition of cell lysis by said coronavirus.
Embodiment 8: The compound for use of any one of embodiments 1 to 7, wherein said treating and/or preventing comprises inhibition of tissue destruction, preferably lung tissue destruction, by said coronavirus.
Embodiment 9: The compound for use of any one of embodiments 1 to 8, wherein said treating and/or preventing comprises administration of the compound, pharmaceutically acceptable salt, or prodrug to the lung or parts thereof, preferably by inhalation.
Embodiment 10: The compound for use of any one of embodiments 1 to 9, wherein said treating and/or preventing comprises oral administration of the compound, pharmaceutically acceptable salt, or prodrug.
Embodiment 11: The compound for use of any one of embodiments 1 to 10, wherein said treating and/or preventing comprises administration of the administration of the compound, pharmaceutically acceptable salt, or prodrug as a tablet or capsule.
Embodiment 12: The compound for use of any one of embodiments 1 to 11, wherein said treating and/or preventing comprises parenteral administration of the compound, pharmaceutically acceptable salt, or prodrug.
Embodiment 13: The compound for use of any one of embodiments 1 to 12, wherein said treating and/or preventing comprises intravenous administration of the compound, pharmaceutically acceptable salt, or prodrug, preferably by continuous infusion.
Embodiment 14: The compound for use of any one of embodiments 1 to 13, wherein said subject is a human.
Embodiment 15: A composition comprising a compound selected from the compounds indicated in Table 1, pharmaceutically acceptable salts, and prodrugs thereof for use in treating and/or preventing a coronavirus infection in a subject.
Embodiment 16: The composition of embodiment 15, wherein said composition is a pharmaceutical composition.
Embodiment 17: A method of treating and/or preventing a coronavirus infection in a subject, comprising administering a compound selected from the compounds indicated in Table 1, pharmaceutically acceptable salts, and prodrugs thereof to said subject, thereby treating and/or preventing said coronavirus infection in said subject.
Embodiment 18: A method of inhibiting coronavirus replication in a cell or tissue, comprising contacting said cell or tissue with a compound selected from the compounds indicated in Table 1, pharmaceutically acceptable salts, and prodrugs thereof.
Embodiment 19: The method of embodiment 18, wherein said method is an in vitro method.
Embodiment 20: Use of a compound selected from the compounds indicated in Table 1, pharmaceutically acceptable salts, and prodrugs thereof for the manufacture of a medicine for treating and/or preventing a coronavirus infection in a subject.
Embodiment 21: A kit comprising a compound or a composition of any one of any one of embodiments 1 to 16 comprised in a housing.
Embodiment 22: The kit of embodiment 21, further comprising a diluent and/or a means of administration.
Embodiment 23: A device comprising a compound selected from the compounds indicated in Table 1, pharmaceutically acceptable salts, and prodrugs thereof.
Embodiment 24: The device of embodiment 23, wherein said device is a means of administration.
Embodiment 25: The device of embodiment 24, wherein said means of administration is an inhaler, a ventilator, an intravenous infusion device, or a syringe, preferably is an inhaler or a ventilator.

All references cited in this specification are herewith incorporated by reference with respect to their entire disclosure content and the disclosure content specifically mentioned in this specification.

The following Examples shall merely illustrate the invention. They shall not be construed, whatsoever, to limit the scope of the invention.

The inventors have also tested the indicated compounds, pharmaceutically acceptable salts and hydrate forms of the above compounds and have confirmed that these also maintain the activity against the virus. The experimental results obtained by the inventors and described in the Experimental Section below demonstrate that all these compounds are useful for the treatment of a SARS-Cov-2 infection in a subject.

### Example 1: Computer-Aided Drug Design

The EXSCALATE platform, the most powerful computing resources currently based in Europe to empower smart in-silico drug design (co-funded by the H2020-FET-HPC ANTAREX project), was exploited to perform High Performance Computing (HPC) simulations, with the final aim to select molecules active against the SARS-CoV-2 virus. The crystal structures of the main functional units of SARS-CoV-2 proteome were obtained from the Protein Data Bank; Table 2 reports the list of the proteins analysed, with the corresponding PDB codes. Homology models of the proteins for which the crystal structure is not available were generated and used. In particular the SARS-CoV-2 proteins most interesting as target to identify antiviral drugs, were selected.

**Table 2: SARS-CoV-2 proteins analyzed and their PDB accession numbers.**

| **Proteins** | **PDB code** |
|---|---|
| 3CL protease | 6LU7 |
| N-protein | 6VYO |
| NSP3 | 6W02 |
| NSP6 | De novo model |
| NSP9 | 6W4B |
| NSP12 | 7BV2 |
| NSP13 | Homology Model |
| NSP14 | Homology Model |
| NSP15 | 6W01 |
| NSP16 | 6W4H |
| PL protease | 6W9C |
| Spike-ACE2 | 6M0J |

Molecular dynamics simulations on the SARS-CoV-2 proteins were performed to explore the conformational space of their active sites, and select several protein conformations particularly suitable for docking simulations. MD simulations were carried out on the protein structures, prepared ad hoc in order to optimize the 3D structure from a chemical and conformational point of view. The structure was firstly subjected to a cycle of energy minimization by steepest descent methods to eliminate all initial steric clashes and obtain a pre-equilibrated model to start from. Then a 100 ps restrained MD simulation (typically 1000 KJ/mole force constant) was performed on the solvent atoms to equilibrate water molecules keeping the solute restrained. Finally, a production run was performed to generate a 1microsecond trajectory with a total of 20.000 collected. Post HPC-run analysis of the results was performed.

High Performance Computing (HPC) simulation was conducted to virtual screen against the SARS-CoV-2 proteins, the Safe in Man (SIM) library, containing commercialized and under development drugs, already proved safe in man (> 10.000 drugs), and the Fraunhofer's BROAD Repurposing Library, containing 5400 marketed drugs and clinical stage compounds and molecules with known mode of action. Duplicates between the libraries were removed before ligand preparation, where all compounds were converted to 3D and prepared with Schrödinger's LigPrep tool. This process generated multiple states for stereoisomers, tautomers, ring conformations (1 stable ring conformer by default) and protonation states. In particular, another Schrödinger package, Epik, was used to assign tautomers and protonation states that would be dominant at a selected pH range (pH=7±1). Ambiguous chiral centers were enumerated, allowing a maximum of 32 isomers to be produced from each input structure. Then, an energy minimization was performed with the OPLS3 force.

The simulation was performed using LiGen^{™} (Ligand Generator), the de novo structure based virtual screening software, designed and developed to run on HPC architectures, which represent the most relevant tool of the EXSCALATE platform. LiGen^{™} is formed by a set of tools that can be combined in a user-defined manner to generate project centric workflows. In particular, LiGenDock is a docking module using LiGenScore to compute the scoring function and the LiGenPass and LiGenPocket modules to obtain the 3D structure of the binding site. Both the docking algorithm implemented in LiGen^{™}, the pharmacophoric docking (LiGenPh4) and the geometrical docking (LiGenGeodock), as well as the different scoring functions calculated, were used in this study to explore different protocols of Virtual Screening (VS) and select the best one in terms of performance.

Docking settings were validated through either redocking (where possible) or docking of known ligands. When tautomers were involved, only the one, with the best docking score, was chosen. The docking score values that predict the binding affinity of the molecules in the protein binding site, are reported (the higher, the better).

Virtual screening was performed on the main SARS-CoV-2 proteins to evaluate the potential poly-pharmacological effect of the compounds on the COVID-19 virus. A total score, corresponding to the sum of the docking scores obtained for each protein, was also calculated for each molecule and used to prioritize the most interesting molecules. Table 3 below reports the results obtained for the best scored molecules, selected for further validation of the antiviral activity in in vitro experiments.

### Example 2: In vitro screening of activity against SARS-CoV-2

The compounds selected as active by the CADD analysis (Example 1) were tested in a SARS-CoV 2 VeroE6-EGFP HTS antiviral Assay. This assay is based on the monitoring of cytopathic effects induced by viral infection of VeroE6 cells constitutively expressing an enhanced green fluorescent protein (EGFP). In details, the VeroE6-EGFP reporter cell line was received from JNJ whereas the SARS-CoV-2 used for infections is a Belgian strain (BetaCov/Belgium/GHB-03021/2020) present in the KU Leuven laboratories.

The test compounds were dissolved at 10mM in dimethylsulphoxide (DMSO) and then diluted in cell culture medium to the required final concentrations. DMSO final concentration was below 0.5%. Then, compounds were mixed with with 0.01 MOI of the virus 8000 VeroE6-EGFP cells/well in 384-well plates. Cells infected by Sars-CoV 2 and treated with Remdesivir 20uM were used as positive control (inhibition 100%) whereas cells infected by Sars-CoV 2 without Remdesivir as negative control (0% inhibition).

After incubation at 37°C for 5 days the EGFP signal of each well was recorded using an argon laser-scanning microscope. Standard whole-well fluorescence plate readout was performed (self-optimizing protocol, ∼6min per 384-well plate, 4 reads/well) and the fluorescence total intensity was measured for both test compounds and control wells.

High-content imaging readout was also performed using a 5x objective, allowing to capture almost the entire well of a 384-well plate at once (auto-focus on each well, no binning, 1 channel). Two values were obtained from high content imaging:
- % Confluence: indicates the increase in confluence of VEROE6-EGFP cells in the test compound wells compared to the control wells. This parameter is based on the quantification of the total surface of the field that gives a green fluorescent signal, due to the presence of EGPF-positive cells in SARS-CoV-2 infected wells treated with test compounds compared to untreated control wells: higher values mean that there are a large number of cells on the microtiter plate bottom surface, small values mean that most of the fluorescence is lost (i.e. the cells died). In the table attached, we reported the Maximum confluence reached (%) after test compound treatment of SARS-CoV-2 infected cells.
- IC50 (µM): indicates the half maximal concentration able to recover the cytopathic effect of infection and is a measure of the potency of a substance in inhibiting viral included cell death. It was determined from dose -response curves obtained testing the compounds antiviral effect at 8 different concentrations.

The results obtained for the compounds tested are shown in Table 4 below.

All the test compounds are able to inhibit virus replication and reduce cytopathic effects of the virus compared to the control, in particular, they recover cell growth at least to 30% of cell confluence. Interestingly, 50 of the 83 selected compounds (60%) are able to recover more than 50% of cell confluence.

The research leading to the results described herein were funded by the European Union.

### Literature:

- Anand et al., Science 2003;300(5626): 1763-7
- Forster et al., PNAS 2020 117 (17) 9241-9243
- Holmes, K.V. et al., 1996. Virology 1, 1075-1093
- O'Conner and Roth, Nat Rev Drug Disc 2005: 4:1005-1014
- Pereira, H et al., 1989 Coronaviridae. Andrewes 'Viruses of Vertebrates, 5th ed.pp. 42-57
- Wu et al., Acta Pharm Sin B. 2020; 10(5): 766-788

**Table 3: LiGen docking score results**

| **No.** | **Compound (Current) Name** | **3CL** | **N-prot** | **NSP 3** | **NSP 6** | **NSP 9** | **NSP 12** | **NSP 13** | **NSP 14** | **NSP 15** | **NSP 16** | **Plpro** | **Spike-ACE2** | **Total sco re** |
|---|---|---|---|---|---|---|---|---|---|---|---|---|---|---|
| 1 | METHOTREXATE | 4.00 | 4.34 | 4.32 | 4.35 | 3.50 | 4.18 | 4.47 | 5.77 | 4.53 | 5.82 | 4.48 | 5.12 | 54.88 |
| 2 | Trimetrexate | 5.04 | nd | nd | 4.26 | 4.06 | 4.82 | nd | nd | 4.39 | 5.59 | 4.54 | nd | 32.70 |
| 3 | Pralatrexate | 4.56 | 4.38 | 4.06 | 5.26 | 3.93 | 4.52 | 4.87 | 4.10 | 5.48 | 4.72 | 4.77 | 4.83 | 55.49 |
| 4 | CH-4051 | 4.73 | nd | 4.88 | 5.57 | 3.82 | 4.27 | 5.31 | nd | 4.76 | 5.14 | 4.64 | nd | 43.13 |
| 5 | CH-1504 | 4.27 | 4.83 | 5.05 | 5.11 | 3.80 | 4.47 | n.d. | nd | 5.14 | 5.63 | 5.15 | 4.63 | 48.08 |
| 6 | L-MDAM | 5.59 | nd | 4.59 | 5.32 | 3.99 | 4.40 | 4.95 | nd | 4.41 | 5.57 | 4.53 | nd | 43.35 |
| 7 | Talotrexin | 5.23 | nd | nd | 5.38 | 4.07 | 4.75 | nd | nd | 4.93 | nd | 4.71 | 5.33 | 34.39 |
| 8 | Edatrexate | 5.80 | nd | 4.90 | 5.21 | 3.58 | 4.50 | nd | nd | 4.58 | 5.68 | 4.85 | nd | 39.09 |
| 9 | MX-68 | 4.92 | 4.81 | 4.46 | 4.90 | 4.13 | 5.33 | 4.32 | nd | 5.15 | 5.57 | 4.74 | nd | 48.33 |
| 10 | Aminopterin | 4.71 | 4.29 | 3.98 | 4.53 | 3.73 | 4.20 | 4.63 | 6.01 | 4.86 | 5.45 | 4.21 | 5.59 | 56.20 |
| 12 | 10-Deazaaminopterin | 4.22 | nd | 4.97 | 4.88 | 4.43 | 4.40 | 4.75 | nd | 5.22 | 5.30 | 4.78 | 5.52 | 48.46 |
| 13 | Setanaxi b | 5.10 | 4.79 | 5.10 | 5.13 | 4.77 | 5.02 | 5.16 | 5.67 | 4.49 | 4.66 | 5.44 | 5.70 | 61.04 |
| 14 | 640486 | 4.88 | 5.40 | 5.07 | 5.17 | 4.81 | 4.66 | 5.18 | 6.11 | 4.89 | 4.90 | 4.16 | 5.06 | 60.31 |
| 15 | 640491 | 5.67 | 4.70 | 4.80 | 5.42 | 4.88 | 4.72 | nd | 6.59 | 5.19 | 5.70 | 4.73 | 5.26 | 57.67 |
| 16 | 640493 | 4.80 | 4.81 | 5.70 | 5.48 | 4.54 | 4.88 | 5.06 | nd | 5.46 | 5.63 | 4.81 | nd | 51.17 |
| 17 | 640497 | 5.22 | 5.07 | nd | 5.29 | 4.67 | 4.75 | n.d. | nd | 5.23 | nd | nd | nd | 30.23 |
| 18 | 640498 | 4.60 | 4.78 | 4.61 | 4.60 | 4.04 | 4.45 | 5.08 | 6.18 | n.d. | 5.03 | 4.81 | 4.80 | 52.97 |
| 19 | GKT-136901 | 5.36 | 4.93 | 5.77 | 5.50 | 4.10 | 4.67 | nd | 6.52 | 5.47 | 5.58 | 5.14 | nd | 53.04 |
| 20 | PIK-III | 4.83 | 4.67 | 5.29 | 5.16 | 4.10 | 4.46 | 4.27 | nd | 5.78 | 5.16 | 4.82 | 4.61 | 53.16 |
| 21 | 781398 | 4.94 | 5.04 | 4.94 | 4.83 | 4.14 | 4.54 | 5.25 | nd | 5.00 | 5.09 | 5.02 | 5.02 | 53.80 |
| 22 | 781406 | 4.75 | 5.21 | 5.46 | 4.91 | 3.95 | 4.70 | 5.06 | 6.21 | 5.40 | 5.04 | 5.23 | nd | 55.91 |
| 23 | 781408 | 4.97 | 4.91 | 5.78 | 4.90 | 3.87 | 4.32 | 5.23 | nd | 5.41 | 5.24 | 4.44 | 5.05 | 54.13 |
| 24 | 781410 | 5.45 | 5.36 | 6.00 | 5.22 | 3.95 | 4.82 | 5.24 | 6.89 | 5.28 | 5.57 | 5.21 | 5.84 | 64.84 |
| 25 | 781424 | 4.83 | 4.68 | 5.28 | 5.15 | 4.31 | 4.30 | 4.85 | 5.86 | 5.14 | 5.29 | 5.03 | 4.66 | 59.36 |
| 26 | Arotinoid acid | 5.56 | 5.69 | 5.45 | 4.45 | 5.20 | 5.30 | 5.39 | 7.16 | 5.50 | 5.68 | 5.51 | 6.51 | 67.38 |
| 27 | Palovarotene | 5.56 | 5.66 | 5.56 | 4.70 | 5.28 | 5.14 | 5.04 | 6.20 | 4.65 | 6.01 | 6.21 | 6.23 | 66.24 |
| 28 | Ro-136298 | 5.49 | 4.68 | nd | 5.52 | 4.72 | 5.48 | 5.68 | nd | 5.51 | 6.74 | 5.32 | nd | 49.14 |
| 29 | Etarotene | 5.58 | nd | nd | 5.52 | 4.65 | 5.15 | nd | nd | 5.63 | nd | 5.56 | nd | 32.10 |
| 30 | Mofarotene | 5.19 | 5.78 | 5.64 | 5.50 | 5.39 | 5.36 | nd | nd | 5.34 | 5.40 | 4.90 | nd | 48.50 |
| 32 | WAY-600 | 4.54 | 4.97 | 5.91 | 5.78 | 4.91 | 5.04 | 4.57 | 5.41 | 5.50 | 5.69 | 5.23 | 5.65 | 63.19 |
| 33 | CFI-400945 | 6.05 | 5.75 | nd | 5.58 | 4.48 | 5.48 | nd | nd | 5.85 | nd | 5.72 | nd | 38.92 |
| 34 | VE-822 (Berzosertib) | 4.27 | 5.31 | 4.95 | 4.33 | 4.09 | 5.00 | 4.71 | 5.41 | 4.80 | 6.65 | 4.90 | 6.21 | 60.63 |
| 35 | Tandutinib | 4.65 | 4.84 | 4.47 | 4.51 | 5.17 | 4.49 | 4.07 | 5.10 | 5.08 | 3.92 | 4.93 | 5.83 | 57.08 |
| 36 | Bemesetron | 5.02 | 4.74 | 5.36 | 4.79 | 3.89 | 4.47 | 4.53 | 5.83 | 4.76 | 5.06 | 5.11 | 4.67 | 58.24 |
| 37 | Eltrombopag | 5.53 | 5.29 | 5.75 | 4.78 | 4.81 | 4.89 | 4.59 | 5.44 | 4.60 | 5.13 | 5.64 | 6.05 | 62.49 |
| 39 | Verteporfin | 4.50 | 4.26 | 4.57 | 4.25 | 4.80 | 5.39 | 4.29 | 3.41 | 5.36 | 5.83 | 4.96 | 5.73 | 57.35 |
| 41 | Metoprine | 4.97 | 4.43 | 5.04 | 5.20 | 4.79 | 4.40 | 4.89 | 5.76 | 5.04 | 4.78 | 4.73 | 4.92 | 58.95 |
| 42 | Pyrimethamine | 4.47 | 4.67 | 4.27 | 3.92 | 4.56 | 4.42 | 4.47 | 5.88 | 5.59 | 4.64 | 4.55 | 4.38 | 55.81 |
| 43 | MZPES | 4.94 | 5.49 | 4.64 | 4.70 | 3.82 | 4.46 | 4.66 | 6.06 | 4.96 | 4.93 | 5.24 | 4.47 | 58.37 |
| 44 | Sipatrigine | 5.07 | 4.92 | 4.90 | 6.31 | 4.69 | 4.63 | 4.97 | 6.12 | 5.65 | 5.01 | 5.05 | 5.42 | 62.75 |
| 45 | BW-4030W92 | 4.80 | 4.88 | 5.10 | 4.85 | 4.33 | 4.32 | 4.90 | 5.65 | 5.27 | 5.31 | 4.82 | 4.62 | 58.84 |
| 47 | brefeldin-a | 4.95 | 4.69 | 4.50 | 5.35 | 3.96 | 4.77 | 4.64 | 5.28 | 4.93 | 5.67 | 5.14 | 5.13 | 59.01 |
| 48 | BS-181 | 4.77 | 5.13 | 5.16 | 5.02 | 3.72 | 4.47 | 3.94 | 5.40 | 5.52 | 5.33 | 4.27 | 5.19 | 57.93 |
| 51 | MCOPPB | 5.60 | 5.15 | 5.07 | 4.61 | 4.25 | 4.76 | 4.74 | 4.72 | 5.16 | 5.53 | 5.19 | 5.18 | 59.95 |
| 52 | MG-132 | 5.55 | 5.00 | 4.26 | 4.86 | 4.52 | 4.63 | 4.55 | 4.67 | 5.49 | 5.26 | 4.51 | 5.22 | 58.51 |
| 53 | PHA-665752 | 4.65 | 5.29 | 4.95 | 5.05 | 4.71 | 5.34 | 5.66 | 5.54 | 4.84 | 5.63 | 5.48 | 6.48 | 63.62 |
| 54 | SAR405 | 5.25 | 4.79 | 4.24 | 5.72 | 4.11 | 4.60 | 4.66 | 5.04 | 5.48 | 5.20 | 5.13 | 5.20 | 59.42 |
| 56 | VU-0364739 | 5.73 | 4.46 | 5.13 | 4.70 | 4.82 | 5.05 | 5.25 | 6.78 | 4.88 | 5.40 | 5.07 | 5.83 | 63.10 |
| 57 | ADOMEGLIVANT | nd | nd | 5.27 | 5.64 | 4.14 | 5.35 | nd | nd | nd | 5.63 | 5.27 | nd | 31.30 |
| 59 | UNC0646 | 4.55 | 4.91 | 4.69 | 4.60 | 3.91 | 4.94 | 4.93 | 5.91 | 4.96 | 4.48 | 4.91 | 6.04 | 58.82 |
| 60 | VPS34-IN-1 | 4.88 | nd | 5.84 | 5.61 | 4.42 | 5.19 | nd | nd | 5.81 | 6.07 | 4.95 | nd | 42.78 |
| 61 | ND-630 | 5.14 | nd | 5.70 | nd | 3.83 | 5.07 | 4.84 | nd | 5.42 | nd | 4.72 | nd | 34.74 |
| 62 | NVP-BHG712 | 5.37 | 5.30 | 5.50 | 5.00 | 5.14 | 5.34 | 4.89 | 7.60 | 5.11 | 6.13 | 5.87 | 6.94 | 68.20 |
| 63 | Cathepsin Inhibitor 1 | 4.26 | 4.71 | 5.13 | 4.80 | 4.88 | 4.45 | 4.72 | 6.11 | 6.15 | 5.28 | 5.10 | 5.36 | 60.95 |
| 64 | MF63 | 5.77 | 5.88 | 6.47 | 6.39 | 5.17 | 4.99 | 5.45 | nd | 6.08 | 5.90 | 5.36 | nd | 57.45 |
| 65 | CENICRIVIROC | 4.93 | nd | nd | nd | 5.25 | 4.76 | nd | 4.86 | nd | nd | 5.75 | nd | 25.54 |
| 66 | TYRPHOSTIN A9 | 5.34 | 5.31 | 5.23 | 5.48 | 4.26 | 4.68 | 4.86 | 6.02 | 5.55 | 5.48 | 5.18 | 4.95 | 62.34 |
| 67 | GSK2194069 | 4.95 | 5.20 | 5.27 | 4.11 | 4.90 | 5.25 | 4.85 | 7.26 | 5.11 | 6.06 | 5.31 | 5.51 | 63.79 |
| 69 | NU 7441 | 5.73 | 5.49 | 5.42 | 4.54 | 4.27 | 4.99 | 4.46 | 6.69 | 5.64 | 5.50 | 5.45 | 5.36 | 63.54 |
| 70 | CHIR-265 | 5.96 | 5.23 | 4.87 | 4.25 | 4.73 | 5.06 | 4.41 | 7.17 | 5.38 | 6.39 | 5.29 | 5.58 | 64.32 |
| 72 | MMV665852 | 5.16 | 5.40 | nd | 5.60 | 4.68 | 4.97 | 5.13 | 6.10 | 5.29 | 5.68 | 5.08 | nd | 53.09 |
| 73 | PB49673382 | 5.61 | 6.05 | 5.70 | 5.71 | 4.69 | 5.23 | nd | nd | 5.88 | 5.88 | 5.76 | nd | 50.50 |
| 74 | DHCaA | 4.48 | 4.80 | 5.04 | 4.74 | 4.09 | 4.53 | 4.46 | 5.83 | 4.79 | 5.08 | 4.88 | 5.46 | 58.19 |
| 75 | Coumarin 7 | 4.97 | 5.22 | 5.62 | 4.85 | 4.36 | 4.79 | nd | nd | 4.98 | nd | 5.82 | nd | 40.61 |
| 76 | Nexinhib20 | 5.20 | 5.00 | nd | 5.13 | 3.95 | 4.66 | 4.98 | nd | 5.31 | 5.73 | 4.73 | 4.79 | 49.48 |
| 78 | bepridil | 4.58 | 4.45 | 5.68 | 4.44 | 3.93 | 4.12 | 4.18 | 5.84 | 5.01 | 4.50 | 4.79 | 5.10 | 56.62 |
| 79 | Tetrandrine (Fanchinine) | 4.97 | 4.80 | 4.88 | 5.87 | 4.95 | 5.22 | 4.16 | 3.78 | 5.96 | 4.68 | 4.70 | 5.95 | 59.92 |
| 80 | U-18666A | 4.68 | 4.99 | 5.59 | 4.16 | 4.48 | 4.90 | 4.54 | 4.72 | 4.39 | 4.96 | 5.68 | 5.23 | 58.31 |
| 81 | CP-640186 | 5.33 | 4.97 | 4.81 | 4.56 | 4.59 | 5.04 | 4.79 | 5.46 | 4.25 | 5.56 | 5.67 | 5.73 | 60.76 |
| 82 | CLOMIPHENE | 5.74 | 5.06 | 5.69 | 4.84 | 5.07 | 4.59 | 4.52 | 6.83 | 4.55 | 5.61 | 5.46 | 6.32 | 64.27 |
| 84 | TILORONE | 4.67 | 4.24 | 3.84 | 4.22 | 3.79 | 4.49 | 3.54 | 4.05 | 4.14 | 4.08 | 4.35 | 5.46 | 50.86 |
| 85 | STF-62247 | 4.69 | 5.13 | 5.42 | 5.29 | 4.05 | 4.53 | 4.92 | 6.07 | 5.49 | 5.83 | 5.47 | 5.72 | 62.61 |
| 86 | pyrintegrin | 5.27 | 4.62 | 4.63 | 4.56 | 4.58 | 4.63 | 5.26 | 6.76 | 4.74 | 5.44 | 4.94 | 5.75 | 61.17 |
| 87 | Y-134 | 5.39 | 5.47 | 5.28 | 5.52 | 5.62 | 5.27 | 5.04 | 6.37 | 5.30 | 5.94 | 5.90 | 6.90 | 68.01 |
| 89 | Igmesine | 4.49 | 4.74 | 5.84 | 5.61 | 4.65 | 4.66 | 4.09 | 6.40 | 5.85 | 4.13 | 4.71 | 4.82 | 59.98 |
| 90 | Cyclobenzaprine Hydrochloride | 4.51 | 4.66 | 5.87 | 4.88 | 4.37 | 4.40 | 4.87 | 5.63 | 4.90 | 4.94 | 5.35 | 4.44 | 58.82 |
| 92 | GNF-5837 | 6.25 | 6.34 | 5.19 | 4.23 | 4.50 | 5.13 | 4.59 | 5.21 | 5.33 | 5.99 | 5.25 | 6.45 | 64.45 |
| 94 | Trifluoperazine dihydrochloride | 4.64 | 4.52 | 4.53 | 5.66 | 4.19 | 4.12 | 3.90 | 4.37 | 5.43 | 4.62 | 4.29 | 5.77 | 56.05 |
| 95 | UNC0642 | 5.25 | 4.60 | 5.27 | 4.82 | 4.21 | 4.88 | 5.04 | 4.97 | 4.49 | 5.74 | 5.11 | 5.76 | 60.14 |
| 98 | Monensin Sodium | 4.81 | 5.95 | 4.46 | 4.39 | 4.92 | 4.86 | 4.89 | 5.95 | 5.55 | 4.12 | 5.36 | 7.03 | 62.30 |
| 99 | AG1024 | 4.92 | 5.06 | 5.85 | 5.17 | 4.13 | 4.56 | 4.97 | 5.80 | 5.38 | 5.38 | 4.74 | 5.05 | 61.01 |
| 101 | CP-640186 hydrochloride | 5.33 | 4.97 | 4.81 | 4.56 | 4.59 | 5.04 | 4.79 | 5.46 | 4.25 | 5.56 | 5.67 | 5.73 | 60.76 |
| 102 | Bithionol | 4.86 | 4.74 | 5.00 | 4.81 | 4.43 | 4.74 | 4.22 | 5.38 | 5.61 | 5.83 | 4.61 | 4.58 | 58.82 |
| 103 | Tyrphostin 9 | 5.34 | 5.31 | 5.23 | 5.48 | 4.26 | 4.68 | 4.86 | 6.02 | 5.55 | 5.48 | 5.18 | 4.95 | 62.34 |
| 104 | YM201636 | 5.55 | 5.49 | 5.28 | 5.31 | 5.17 | 5.05 | 4.73 | 7.53 | 5.38 | 4.90 | 5.57 | 6.27 | 66.23 |
| 105 | 4E1RCat | 5.61 | 5.37 | 5.60 | 4.97 | 5.28 | 5.07 | 5.64 | 7.36 | 5.03 | 5.79 | 5.19 | 6.83 | 67.74 |
| 108 | Ixazomib | 4.54 | 4.41 | 4.68 | 4.75 | 4.57 | 4.45 | 4.80 | 5.80 | 5.68 | 4.30 | 4.82 | 5.29 | 0.00 |
| 109 | thiothixene | 4.02 | 5.40 | 4.07 | 4.33 | 4.40 | 4.09 | 4.46 | 5.82 | 5.68 | 4.57 | 4.59 | 5.47 | 56.91 |

**Table 4:**

| **N.** | **Compound (current) Name** | **IC50 (µM)** | **Maximum confluence reached (%)** |
|---|---|---|---|
| 1 | METHOTREXATE | 0.05 | 64 |
| 2 | Trimetrexate | 0.33 | 78.23 |
| 3 | Pralatrexate | 0.01 | 76.65 |
| 10 | Aminopterin | 0.35 | 72.54 |
| 13 | Setanaxi b | 7.57 | 101.52 |
| 20 | PIK-III | 7.92 | 98.17 |
| 26 | Arotinoid acid | 33.71 | 105.73 |
| 32 | WAY-600 | 9.43 | 67.69 |
| 33 | CFI-400945 | 11.91 | 105.46 |
| 34 | VE-822 (Berzosertib) | 2.67 | 99.21 |
| 35 | Tandutinib | 4.23 | 80.09 |
| 36 | bemesetron | 43.9 | 69.05 |
| 37 | Eltrombopag | 58.08 | 63.17 |
| 39 | verteporfin | 30 | 89.5 |
| 41 | Metoprine | 2.34 | 58.02 |
| 47 | brefeldin-a | 0.02 | 121.63 |
| 48 | BS-181 | 12.93 | 47.31 |
| 51 | MCOPPB | 13.14 | 57.77 |
| 52 | MG-132 | 0.32 | 82.81 |
| 53 | PHA-665752 | 4.97 | 95.69 |
| 54 | SAR405 | 10 | 89 |
| 56 | VU-0364739 | 11.64 | 98.46 |
| 57 | ADOMEGLIVANT | 10.55 | 39.53 |
| 59 | UNC0646 | 12.03 | 80.08 |
| 60 | VPS34-IN-1 | 0.4 | 45.62 |
| 61 | ND-630 | 0.38 | 78.18 |
| 62 | NVP-BHG712 | 5.26 | 88.43 |
| 63 | Cathepsin Inhibitor 1 | 8.98 | 96.18 |
| 64 | MF63 | 13.29 | 98.48 |
| 65 | CENICRIVIROC | 55 | 70.92 |
| 66 | TYRPHOSTIN A9 | 0.83 | 47.6 |
| 67 | GSK2194069 | 16.98 | 31.06 |
| 69 | NU 7441 | 10 | 90.45 |
| 70 | CHIR-265 | 14.64 | 44.1 |
| 72 | MMV665852 | 3.97 | 45.87 |
| 73 | PB49673382 | 6.42 | 53.9 |
| 74 | DHCaA | 3.69 | 37.98 |
| 75 | Coumarin 7 | 0.36 | 33.14 |
| 76 | Nexinhib20 | 4.33 | 50.44 |
| 78 | bepridil | 3.86 | 30.41 |
| 79 | Tetrandrine (Fanchinine) | 11.66 | 99.64 |
| 80 | U-18666A | 1.15 | 44.66 |
| 81 | CP-640186 | 3.78 | 51 |
| 82 | CLOMIPHENE CITRATE | 13.62 | 54.73 |
| 84 | TILORONE | 16.44 | 68.89 |
| 85 | STF-62247 | 4.11 | 47.81 |
| 86 | pyrintegrin | 1.31 | 55.04 |
| 87 | Y-134 | 29.87 | 41.04 |
| 89 | Igmesine | 11.2 | 58.34 |
| 90 | Cyclobenzaprine Hydrochloride | 12.1 | 70,19 |
| 92 | GNF-5837 | 5.23 | 75.5 |
| 94 | Trifluoperazine dihydrochloride | 16.65 | 37.97 |
| 95 | UNC0642 | 16.73 | 31.06 |
| 98 | Monensin Sodium | 3.18 | 38.24 |
| 99 | AG1024 | 7.23 | 37.27 |
| 101 | CP-640186 hydrochloride | 3.78 | 50.88 |
| 102 | Bithionol | 19.1 | 31.25 |
| 103 | Tyrphostin 9 | 204.65 | 51.51 |
| 104 | YM201636 | 4.73 | 35.48 |
| 105 | 4E1RCat | 236.34 | 52.15 |
| 108 | Ixazomib | 135.08 | 30.79 |
| 109 | thiothixene | 18.24 | 36.55 |

## Claims

1. A compound selected from the compounds indicated in Table 1 and pharmaceutically acceptable salts and prodrugs thereof for use in treating and/or preventing a coronavirus infection in a subject.

2. The compound for use of claim 1, wherein said compound, pharmaceutically acceptable salt, or prodrug is comprised in a pharmaceutical composition.

3. The compound for use of claim 1 or 2, wherein said coronavirus is severe acute respiratory syndrome coronavirus (SARS-CoV)-2, SARS-CoV-1, or Middle East respiratory syndrome coronavirus (MERS-CoV).

4. The compound for use of any one of claims 1 to 3, wherein said coronavirus is SARS-CoV-2.

5. The compound for use of any one of claims 1 to 4, wherein said treating and/or preventing comprises inhibition of coronavirus replication, inhibition of cell lysis by said coronavirus, and/or inhibition of tissue destruction, preferably lung tissue destruction, by said coronavirus.

6. The compound for use of any one of claims 1 to 5, wherein said treating and/or preventing comprises
(i) administration of the compound, pharmaceutically acceptable salt, or prodrug to the lung or parts thereof, preferably by inhalation;
(ii) oral administration of the compound, pharmaceutically acceptable salt, or prodrug,
(iii) administration of the administration of the compound, pharmaceutically acceptable salt, or prodrug as a tablet or capsule,
(iii) parenteral administration of the compound, pharmaceutically acceptable salt, or prodrug,
(iv) intravenous administration of the compound, pharmaceutically acceptable salt, or prodrug, preferably by continuous infusion, or
(v) any combination of (i) to (iv).

7. The compound for use of any one of claims 1 to 6, wherein said subject is a human.

8. The compound for use of any one of claims 1 to 7, wherein said compound is selected from the compounds indicated in Table 1 and pharmaceutically acceptable salts thereof.

9. A composition comprising a compound selected from the compounds indicated in Table 1, pharmaceutically acceptable salts, and prodrugs thereof for use in treating and/or preventing a coronavirus infection in a subject.

10. An in vitro method of inhibiting coronavirus replication in a cell or tissue, comprising contacting said cell or tissue with a compound selected from the compounds indicated in Table 1, pharmaceutically acceptable salts, and prodrugs thereof.

11. A kit comprising a compound or a composition of any one of any one of claims 1 to 8 comprised in a housing.

12. The kit of claim 11, further comprising a diluent and/or a means of administration.

13. A device comprising a compound selected from the compounds indicated in Table 1, pharmaceutically acceptable salts, and prodrugs thereof.

14. The device of claim 13, wherein said device is a means of administration.

15. The device of claim 14, wherein said means of administration is an inhaler, a ventilator, an intravenous infusion device, or a syringe, preferably is an inhaler or a ventilator.
